(19) Europäisches Patentamt
European Patent Office
Office européen des brevets

(11) EP 2 904 531 B1

(12) **EUROPÄISCHE PATENTSCHRIFT**

(45) Veröffentlichungstag und Bekanntmachung des Hinweises auf die Patenterteilung:
**19.12.2018 Patentblatt 2018/51**

(21) Anmeldenummer: **13766556.8**

(22) Anmeldetag: **27.09.2013**

(51) Int Cl.:
***G06F 19/00*** *(2018.01)* ***G03H 1/00*** *(2006.01)*
***G03H 1/02*** *(2006.01)*

(86) Internationale Anmeldenummer:
**PCT/EP2013/070163**

(87) Internationale Veröffentlichungsnummer:
**WO 2014/053408 (10.04.2014 Gazette 2014/15)**

# (54) AUSWAHLVERFAHREN FÜR PHOTOINITIATORSYSTEME

METHOD FOR SELECTING PHOTOINITIATOR SYSTEMS

PROCEDE DE SELECTION POUR DES SYSTÈMES PHOTOAMORCEURS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.10.2012 EP 12187040**

(43) Veröffentlichungstag der Anmeldung:
**12.08.2015 Patentblatt 2015/33**

(73) Patentinhaber: **Covestro Deutschland AG**
**51373 Leverkusen (DE)**

(72) Erfinder:
- **RÖLLE, Thomas**
**51381 Leverkusen (DE)**
- **BERNETH, Horst**
**51373 Leverkusen (DE)**
- **BRUDER, Friedrich-Karl**
**47802 Krefeld (DE)**
- **FÄCKE, Thomas**
**51375 Leverkusen (DE)**
- **WEISER, Marc-Stephan**
**51377 Leverkusen (DE)**
- **HÖNEL, Dennis**
**53909 Zülpich-Wichterich (DE)**
- **DIEDRICH, Christian**
**40591 Düsseldorf (DE)**

(74) Vertreter: **Levpat**
**c/o Covestro AG**
**Gebäude 4825**
**51365 Leverkusen (DE)**

(56) Entgegenhaltungen:
**US-A1- 2008 145 790 US-A1- 2012 237 856**

- **FOUASSIER J. P. ET AL: "Photopolymerization reactions under visible lights: principle, mechanisms and examples of applications", PROGRESS IN ORGANIC COATINGS, Bd. 47, Nr. 1, 1. Juli 2003 (2003-07-01), Seiten 16-36, XP055072106, ISSN: 0300-9440, DOI: 10.1016/S0300-9440(03)00011-0**
- **MERAL A. ET AL: "Mechanistic Study of Photoinitiated Free Radical Polymerization Using Thioxanthone Thioacetic Acid as One-Component Type II Photoinitiator", MACROMOLECULES, Bd. 38, Nr. 10, 1. Mai 2005 (2005-05-01), Seiten 4133-4138, XP055069778, ISSN: 0024-9297, DOI: 10.1021/ma047560t**
- **WEISS P.: "Photo-induced polymerization", PURE AND APPLIED CHEMISTRY, Bd. 15, Nr. 3-4, 1. Januar 1967 (1967-01-01), Seiten 587-600, XP055070476, ISSN: 0033-4545, DOI: 10.1351/pac196715030587**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 904 531 B1

Printed by Jouve, 75001 PARIS (FR)

## Beschreibung

Die Erfindung betrifft ein Verfahren zur Auswahl von Photoinitiatorsystemen, umfassend mindestens einen Sensibilisator und mindestens einen Coinitiator, für Photopolymer-Formulierungen zur Herstellung von holographischen Medien.

Photopolymer-Formulierungen der eingangs genannten Art sind im Stand der Technik bekannt. So ist beispielsweise in der WO 2008/125229 A1 eine Photopolymer-Formulierung beschrieben, die eine Polyol-Komponente, eine Polyisocyanat-Komponente, ein Schreibmonomer auf Acrylatbasis sowie Photoinitiatoren enthaltend einen Coinitiator und einen Farbstoff umfasst. Im ausgehärtetem Zustand sind in der aus Polyol- und Polyisocyanat-Komponente gebildeten Polyurethanmatrix das Schreibmonomer und die Photoinitiatoren räumlich isotrop verteilt eingebettet.

Für die Verwendungen von Photopolymer-Formulierungen spielt die durch die holographische Belichtung im Photopolymer erzeugte Brechungsindexmodulation $\Delta n$ die entscheidende Rolle. Bei der holographischen Belichtung wird das Interferenzfeld aus Signal- und Referenzlichtstrahl (im einfachsten Fall das zweier ebener Wellen) durch die lokale Photopolymerisation von z.B. hochbrechenden Acrylaten an Orten hoher Intensität im Interferenzfeld in ein Brechungsindexgitter abgebildet. Das Brechungsindexgitter im Photopolymeren (das Hologramm) enthält alle Information des Signallichtstrahls. Durch Beleuchtung des Hologramms nur mit dem Referenzlichtstrahl kann dann das Signal wieder rekonstruiert werden. Die Stärke des so rekonstruierten Signals im Verhältnis zur Stärke des eingestrahlten Referenzlichts wird Beugungseffizienz, im Folgenden DE wie Diffraction Efficiency, genannt.

Im einfachsten Fall eines Hologramms, das aus der Überlagerung zweier ebener Wellen entsteht ergibt sich die DE aus dem Quotienten der Intensität des bei der Rekonstruktion abgebeugten Lichtes und der Summe der Intensitäten aus eingestrahltem Referenzlicht und abgebeugtem Licht. Je höher die DE desto effizienter ist ein Hologramm in Bezug auf die Lichtmenge des Referenzlichtes die notwendig ist, um das Signal mit einer festen Helligkeit sichtbar zu machen.

Bei Beleuchtung des Hologramms mit z.B. weißem Licht hängt die Breite des spektralen Bereiches, der zur Rekonstruktion des Hologramms beitragen kann, ebenfalls nur von der Schichtdicke d ab. Dabei gilt: je kleiner d desto größer die jeweiligen Akzeptanzbreiten. Will man daher helle und leicht sichtbare Hologramme herstellen, ist ein hohes $\Delta n$ und eine geringe Dicke d anzustreben und zwar so, dass DE möglichst groß wird. Das heißt, je höher $\Delta n$ wird, desto mehr Freiraum zur Gestaltung der Schichtdicke d für helle Hologramme erreicht man ohne Verlust an DE. Daher kommt der Optimierung von $\Delta n$ bei der Optimierung von Photopolymer-Formulierungen eine herausragende Bedeutung zu (P. Hariharan, Optical Holography, 2nd Edition, Cambridge University Press, 1996).

Um ein möglichst hohes $\Delta n$ und DE bei Hologrammen realisieren zu können, sollten grundsätzlich die Matrixpolymere und die Schreibmonomere einer Photopolymer-Formulierung so gewählt werden, dass sie sich in ihren Brechungsindizes möglichst stark unterscheiden. Eine Möglichkeit zur Realisierung ist, Matrixpolymere mit einem möglichst niedrigen und Schreibmonomere mit einem möglichst hohen Brechungsindex zu verwenden. Geeignete Matrixpolymere mit niedrigem Brechungsindex sind beispielsweise durch Umsetzung einer Polyol- mit einer Polyisocyanat-Komponente erhältliche Polyurethane.

Neben hohen DE- und $\Delta n$- Werten ist es für holographische Medien aus Photopolymer-Formulierungen aber auch von großer Bedeutung, dass die Matrixpolymere im fertigen Medium hoch vernetzt sind. Falls der Vernetzungsgrad zu niedrig ist, weist das Medium keine ausreichende Stabilität auf. Dies kann dazu führen, dass die Qualität von in die Medien eingeschriebenen Hologrammen erheblich vermindert ist. Im schlimmsten Fall können die Hologramme sogar nachträglich zerstört werden.

Weiterhin ist es insbesondere für die großtechnische Herstellung von holographischen Medien aus Photopolymer-Formulierungen von großer Bedeutung, dass das verwendete Photoinitiatorsystem optimal auf die Wellenlänge kommerziell erhältlicher Laser-Lichtquellen abgestimmt ist und mit möglichst hoher Quanteneffizienz die Umwandlung des Lichts in effiziente Kettenstarter-Moleküle ermöglicht.

Es ist jedoch festgestellt worden, dass Medien aus den bekannten Photopolymer-Formulierungen häufig keine ausreichende Wellenlängen-Selektivität aufweisen. Außerdem sind in vielen Fällen selbst bis zur Erreichung der minimal notwendigen DE- und $\Delta n$- Werte lange Belichtungszeiten oder hohe Lichtdosen nötig. Damit kann es bei Medien aus den bekannten Photopolymer-Formulierungen einerseits zu Qualitätsproblemen kommen und andererseits ist mit den längeren Belichtungszeiten ein erheblicher Aufwand bei der großtechnischen Produktion verbunden.

Es existiert eine Vielzahl quantenchemischer Methoden beschrieben, durch welche Energien chemischer Reaktionen abgeschätzt werden kann. Die Auswahl einer Methode für ein gegebenes Problem hängt dabei von den Anforderungen an die Genauigkeit sowie von der zur Verfügung stehenden Rechenzeit ab. Bei sehr genauen Verfahren, wie zum Beispiel dem *Coupled Cluster Singles Doubles* mit störungstheoretischer *Triples* Korrektur CCSD(T) (T. Helgaker, P. J¢rgensen und J, Olsen; Molecular Electronic-Structure Theory; Wiley, New York, 2000), sind auch die Anforderungen an die Rechenzeit sehr hoch. Da der Rechenzeitbedarf solcher Methoden zudem mit der siebten Potenz der Systemgröße ($O(N^7)$) ansteigt, sind sie für größere Moleküle mit mehr als 10 - 15 Schweratomen in der Regel nicht einsetzbar. Anstelle dessen werden dichtefunktionaltheoretische Verfahren (DFT) (R. G. Bak und W. Yang; Density-Functional Theory of Atoms and Molecules; Oxford University Press, Oxford 1989) verwendet, deren deutlich höhere Effizienz aber mit einer Genauigkeitseinbuße einhergeht.

Im Laufe der letzten beiden Jahrzehnte sind sehr viele neue Dichtefunktionale entwickelt worden, welche alle inhärente Stärken und Schwächen aufweisen. Die genaue technische Ausgestaltung einer dichtefunktionaltheoretischen Rechnung muss aus diesem Grund auf das zu untersuchende Problem abgestimmt sein. Im Rahmen des erfindungsgemäßen Verfahrens ist in diesem Zusammenhang zu beachten, dass alle berechneten Vorgänge in flüssiger oder amorpher Phase stattfinden und dass ionische und radikalische Spezies beteiligt sind.

Zur einfachen Beschreibung flüssiger Phasen haben sich in der Quantenchemie Kontinuummodelle wie das *COnductor like Screening MOdel* (COSMO) (A. Klamt und G. Schüürmann; J. Am. Chem. Soc. Perkin Trans II; 1993, 799) durchgesetzt. Im Rahmen dieser Modelle wird die Umgebung eines Moleküls durch ein polarisierbares Kontinuum beschrieben. Insbesondere in Lösungsmitteln mit einer geringen Dichte von Wasserstoffbrückenbindungen können mit diesem Verfahren häufig gute Ergebnisse erhalten werden.

Vielfältige Anwendungen der beschriebenen Verfahren zur Berechnung von Reaktionsenergien sind veröffentlicht (W. Koch und M. C. Holthausen; A Chemist's Guide to Density Functional Theory. Wiley-VHC, Weinheim).

Für die Beschreibung elektronisch angeregter Zustände gilt das oben für die Berechnung von Reaktionsenergien gesagte in deutlich stärkerem Maße: Die Verwendung hochgenauer Methoden ist auf kleine Moleküle beschränkt und kommt daher für Farbstoffmoleküle, wie sie in Photoinitiatorsystemen enthalten sind, nicht in Frage. Aus diesem Grund werden auch in diesem Kontext DFT-Verfahren hier in Form der zeitabhängigen Dichtefunktionaltheorie (TDDFT) (M. E. Casida; Time-Dependent Density Functional Response Theory for Molecules; in: Recent Advances in Density Functional Methods, Vol. 1 (D. P. Chong, Ed.); World Scientific: Singapore; 155-192 (1995)) verwendet. Dabei werden einfache hybrid-Dichtefunktionale verwendet, welche eine rechentechnisch sehr effiziente Anwendung erlauben. Es ist aus der Literatur bekannt, dass diese Verfahren für elektronisch angeregte Zustände große mit der Natur der angeregten Zustände (z.B. $\pi\pi^*$-, Rydberg- oder d-d-Übergänge in Übergangsmetallen) variierende systematische Fehler aufweisen (L. Goerigk und S. Grimme; J. Chem. Phys.; 132, 184103, (2010)). Bewegt man sich bei einer gegebenen Anwendung innerhalb einer Anregungsklasse, dann können die Fehler in den Anregungsenergien häufig durch einen globalen Korrekturterm bereinigt werden. Da die für die Farbigkeit von Farbstoffen verantwortlichen Anregungen nahezu ausschließlich $\pi\pi^*$-artig sind, sind die Voraussetzungen für eine systematische Korrektur gegeben.

Zur Abschätzung der Eignung eines gegebenen Farbstoff/Coinitiatorpaares als Photoinitiatorsystem hat sich in der Literatur die Rehm-Weller Gleichung, Formel (I), (D. Rehm, A. Weller, Ber. Bunsenges. Physik. Chem.; 73, 834, (1969)) durchgesetzt, die in einer vereinfachten Variante wie folgt lautet:

$$\Delta G_{et} = E_{ox} - E_{red} - E_{0,0} + C, \text{(I)}$$

dabei ist:

- $\Delta G_{et}$ die freie Energie des Elektronentransfers,
- $E_{ox}$ das Oxidationspotential des Donors,
- $E_{red}$ das Reduktionspotentials des Akzeptors,
- $E_{0,0}$ die Anregungsenergie des Farbstoffs vom elektronischen Grundzustand in den für die Redoxreaktion relevanten angeregten Zustand und
- C die durch den Elektronentransferprozess induzierte Änderung in der elektrostatischen Energie.

Für $E_{ox}$ und $E_{red}$ werden dabei in der Literatur typischerweise die elektrochemischen Reduktions bzw. Oxidationspotentiale angenommen. $E_{0,0}$ wird durch die sich aus $\lambda_{max}$ ergebende Anregungsenergie des Farbstoffs genähert. C wird in polaren Lösungsmitteln als vernachlässigbar klein angenommen.

Zur direkten Anwendung der Rehm-Weller Gleichung für das rationale Design von Photoinitiatorsystemen ist entsprechend dem oben gesagten die experimentelle Bestimmung von $E_{ox}$, $E_{red}$ und $E_{0,0}$ erforderlich. Ist $\Delta G_{et}$ negativ, so ist das System potentiell als Photoinitiator geeignet. Bei käuflich nicht erhältlichen oder chemisch schwierig zugänglichen Substanzen ist das Verfahren nicht geeignet, da es keine einfache Art und Weise gibt, um die erforderlichen Größen ohne Experiment hinreichend genau abzuschätzen. Darüber hinaus ist anzumerken, dass durch die Verwendung der experimentellen (Singulett) Anregungsenergie als $E_{0,0}$ ein signifikanter Fehler entsteht, da die Initiationsreaktion häufig zwischen dem niedrigsten Triplett Zustand des Farbstoffs und dem Coinitiator stattfindet. Die im UV/Vis gemessene Anregungsenergie entspricht aber einer Singulett $\rightarrow$ Singulett Anregung, welche in der überwiegenden Mehrzahl der Fälle deutlich größer ist als die eigentlich zu berücksichtigende Singulett $\rightarrow$ Triplett Anregungsenergie.

US 2012/237856 offenbart ein Auswahlverfahren für Additive in Photopolymeren. Ziel der vorliegenden Erfindung war es daher ein Verfahren zur Auswahl von Photoinitiatorsystemen, umfassend mindestens einen Sensibilisator und mindestens einen Coinitiator, für Photopolymer-Formulierungen zur Herstellung von holographischen Medien zu

entwickeln, welches es erlaubt auf Basis der chemischen Strukturen, die ein gegebenes Photoinitiatorsystem bilden, dessen Leistungsfähigkeit im Rahmen einer photochemischen Polymerisation vorherzusagen. Dabei sollten quantenchemischen Rechenverfahren eingesetzt werden, welche es erlauben, die elektronischen Zustände der beteiligten Verbindungen, einschließlich des oben beschriebenen Triplett Zustands des Farbstoffs, korrekt zu beschreiben. Diese Aufgabe wird durch ein Computer-implementiertes Verfahren gelöst, bei dem,

a) ein Photoinitiatorsystem, umfassend mindestens einen Sensibilisator und mindestens einen Coinitiator, ausgewählt wird,

b) der Reaktionsmechanismus des Photoinitiatorsystems durch Aufführung der konkreten Reaktionssequenz unter Berücksichtigung der Redox-Eigenschaften der beteiligten Verbindungen aufgestellt wird, welcher den Übergang des Sensibilisators oder der Sensibilisatoren in einen elektronisch angeregten Zustand beziehungsweise elektronisch angeregte Zustände durch Absorption elektromagnetischer Strahlung und in Folgeschritten die als Initiationsreaktion bezeichnete Reaktion des Sensibilisators im elektronisch angeregten Zustand oder der Sensibilisatoren in elektronisch angeregten Zuständen mit dem Coinitiator oder den Coinitiatoren zu mindestens einem Radikal und weiteren, vom jeweiligen Photoinitiatorsystem abhängigen, Produkten, beinhaltet,

c) die dreidimensionale Molekülgeometrien des Sensibilisators oder der Sensibilisatoren, des Coinitiators oder der Coinitiatoren, sowie aller durch den Reaktionsmechanismus definierten Zwischenstufen und Endprodukte der Initiationsreaktion generiert werden und diese dann einer Konformerenanalayse auf Basis eines Kraftfeldverfahrens unterzogen werden,

d) die Molekülgeometrien der Strukturen aus Schritt c) mit der jeweils niedrigsten relativen Kraftfeldenergie quantenchemisch im elektronischen Grundzustand optimiert werden und die absoluten elektronischen Energien der optimierten Strukturen bestimmt werden,

e) die Anregungsenergien und Oszillatorenstärken des elektronischen Absorptionsspektrums des Sensibilisators oder der Sensibilisatoren mit Hilfe des quantenchemischen zeitabhängigen Dichtefunktionaltheorieverfahrens berechnet werden und die Anregungsenergien um ihren systematischen Fehler korrigiert werden,

f) die Molekülgeometrien aller Sensibilisatoren in den bezüglich der Coinitiationsreaktion relevanten, angeregten elektronischen Zuständen auf dichtefunktional-theoretischem Niveau optimiert werden und die absoluten elektronischen Energien bestimmt werden,

g) die Reaktionsenergien alle Teilreaktionen des im Schritt b) aufgestellten Mechanismus berechnet werden, und

h) das Photoinitiatorsystem als geeignet eingestuft wird, wenn die im Schritt e) bestimmten Anregungsfrequenzen in einem $\pm$50 nm Intervall um die für die Belichtung vorgesehene Lichtwellenlänge liegt und eine Oszillatorenstärke größer als 0.2 aufweist und wenn gleichzeitig alle unter g) berechneten Reaktionsenergien negativ sind.

Im Schritt a) wird ein Photoinitiatorsystem ausgewählt, welches mindestens einen Farbstoff und mindestens einen Coinitiator umfasst. Im Schritt b) wird dann der Reaktionsmechanismus bestimmt, nach welchem die Komponenten des Photoinitiatorsystems miteinander reagieren. Allgemein beinhaltet der Reaktionsmechanismus die Absorption elektromagnetischer Strahlung durch mindestens eine Komponente (Sensibilisator) des Photoinitiatorsystems, welche dadurch in einen elektronisch angeregten Zustand überführt wird und die anschließende Redoxreaktion dieser angeregten Verbindung mit mindestens einer weiteren Komponente (Coinitiator), bei welcher ein reaktives Radikal freigesetzt wird. Die Reaktion dieses Radikals mit einem Monomer der Photopolymerformulierung schließt dann die Initiationsreaktion ab.

Im einfachsten Fall für ein mehrkomponentiges Photoinitiatorsystem besteht das Photoinitiatorsystem aus einen Farbstoff und einem Coinitiator. Der Reaktionsmechanismus ergibt sich dann wie folgt:

1) Sensibilisator + *hv* $\rightarrow$ Sensibilisator*
2) Sensibilisator* + Coinitiator $\rightarrow$ Sensibilisator-Radikal + Coinitiator-Radikal
3) Coinitiator-Radikal $\rightarrow$ Coinitiorfragment + Radikal
4) Sensibilisator-Radikal + Coinitiator $\rightarrow$ Sensibilisator-Fragment + Coinitiorfragment

Dabei bezeichnet "Sensibilisator*" einen elektronisch angeregten Zustand des Sensibilisators. Im Rahmen der Initiationsreaktion sind insbesondere der erste angeregte Singulett Zustand ($S_1$), sowie der erste Triplett Zustand ($T_1$) relevant. Für Photoinitiatorsysteme mit mehr als zwei Komponenten wird eine analoge Vorgehensweise beschritten.

Die konkrete Reaktionssequenz ergibt sich dabei aus den Redox-Eigenschaften der beteiligten Verbindungen. Enthält das System beispielsweise einen im angeregten Zustand leicht zu reduzierenden Sensibilisator und mehrere Coinitiatoren, so ist als primärer Initiationsschritt die Reaktion mit einem oxidierbaren Coinitiator anzunehmen, an welche sich dann eine Sekundärreaktion mit einem reduzierbaren Coinitiator anschließt, durch die der Sensibilisators oxidiert und dadurch wieder in seine ursprüngliche Form überführt wird.

Auf Basis des so aufgestellten Reaktionsmechanismus werden im Schritt c) die dreidimensionalen Molekülstrukturen aller in den Teilreaktionen relevanten Edukte, Produkte und Zwischenprodukte generiert und diese dann einer Konformerenanalyse unterzogen. Dies erfolgt beispielsweise mittels eines stochastischen Verfahrens, bei welchem in mehreren aufeinanderfolgenden Schritten alle drehbaren Bindungen eines Moleküls um zufällige Winkel rotiert werden und die resultierenden Strukturen einer Kraftfeldoptimierung unterzogen werden. Alle nach der Kraftfeldenergie oberhalb eines vorher festgelegten Energieschwellenwertes liegenden Konformere werden verworfen.

Die verbleibenden Konformere werden dann im Schritt d) einer quantenchemischen Geometrieoptimierung auf dichtefunktionaltheoretischem (DFT) Niveau unterzogen. Hierzu wird bevorzugt das Becke-Perdew Austausch-Korrelationsfunktional (BP86) (A. D. Becke; Phys. Rev. A; 38, 3098, (1998); J. Perdew; Phys. Rev. B; 33, 8822, (1986); S. H. Vosko, L. Wilk, M. Nusair; Can. J. Phys.; 58, 1200, (1980)) sowie der Ahlrichs Tripel-ζ-Valenz Basissatzes TZVP (A. Schäfer, C. Huber und R. Ahlrichs; J. Chem. Phys.; 100, 5829, (1994)), besonders bevorzugt zusätzlich das *COnductor like Screening MOdel* (COSMO) (A. Klamt und G. Schüürmann; J. Am. Chem. Soc. Perkin Trans II; 1993, 799) verwendet. Die optimierten Geometrien werden dann gegebenenfalls einer Einzelpunktrechnung auf höherem quantenchemischen Niveau, bevorzugt der DFT Methode unter Verwendung des BH-LYP Funktionals (A. D. Becke; J. Chem. Phys.; 98, 1372, (1993)) und des Ahlrichs Tripel- ζ-Valenz Basissatz TZVP, besonders bevorzugt zusätzlich des COSMO Modells unterzogen.

Anschließend werden auf Basis der optimierten Geometrien der Sensibilisatorkonformere zeitabhängige DFT Rechnungen durchgeführt, um die elektronischen Anregungsenergien ($\varepsilon_i$) und Oszillatorenstärken ($f_i$) der Anregungen im relevanten Bereich des elektromagnetischen Spektrums zu berechnen (Schritt e)). Soweit dabei mehrere Konformere berücksichtigt wurden, werden $\varepsilon_i$ und $f_i$ entsprechend der Boltzmann-Gewichte gemittelt, die sich aus den im Schritt d) berechneten Energien ergeben.

Weiterhin werden die, ebenfalls über alle berücksichtigten Konformere gemittelte, Energien des Sensibilisators im für die primäre CoInitiationsreaktion relevanten elektronischen Zustand auf dem gleichen quantenchemischen Niveau ermittelt, welches für die Einzelpunktrechnungen im Schritt d) verwendet wurde (Schritt f)). Die Geometrien der Sensibilisatoren müssen zu diesem Zweck in den jeweiligen elektronischen Zuständen optimiert werden. Dies kann im Falle des niedrigsten Triplettzustandes im Rahmen einer gewöhnlichen DFT Geometrieoptimierung erfolgen. Für den Fall eines höher angeregten Triplettzustands oder des ersten angeregten Singulettzustands ist eine zeitabhängige DFT Rechnung nötig. Die Geometrieoptimierung, sowie eine sich gegebenenfalls anschließende Einzelpunktrechnung erfolgen jeweils auf dem gleichen quantenchemischen Niveau, welches auch unter Schritt d) gewählt wird.

Auf Grundlage der so berechneten Energien können im Schritt g) die Reaktionsenergien alle Teilreaktionen des im Schritt b) aufgestellten Mechanismus berechnet werden.

Im abschließenden Schritt h) wird überprüft ob:

1. Die im Schritt e) bestimmten Anregungsenergien im für die für die geplante Anwendung gewünschten Spektralbereich liegen und die Anregung eine Oszillatorenstärke größer als 0.2 aufweist.
2. Die im Schritt g) bestimmten Reaktionsenergien negativ sind.

Ist dies der Fall, dann wird das Photoinitiatorsystem entsprechend dem erfindungsgemäßen Verfahren als geeignet eingestuft.

Gemäß einer ersten bevorzugten Ausführungsform des erfindungsgemäßen Verfahrens ist vorgesehen, dass in den Schritten d) und f) die quantenchemischen Geometrieoptimierungen mit Hilfe des DFT(BP86/TZVP) Verfahrens vorgenommen und anschließend DFT(BH-LYP/TZVP) Einzelpunktrechnung durchgeführt werden,
Ebenfalls bevorzugt ist, wenn die quantenchemischen Rechnungen unter Verwendung des *COnductor like Screening MOdels* (COSMO) durchgeführt werden.

Im Schritt e) kann insbesondere die zeitabhängige DFT(BH-LYP/TZVP) Methode zur Berechnung der Absorptionsspektren verwendet werden.

In Weiterbildung der Erfindung ist vorgesehen, dass die zeitabhängige DFT Rechnung unter Verwendung des COSMO Modells durchgeführt werden.

Bevorzugt ist auch eine Ausführungsform des erfindungsgemäßen Verfahrens bei dem in den Schritten d) und f) an Stelle nur der Molekülgeometrie mit der niedrigsten Kraftfeldenergie alle Molekülgeometrien in einem Energiefenster von 0-8 kJ/mol berücksichtigt werden und sowohl für die Berechnung des Absorptionsspektrums des Sensibilisators im Schritt e) als auch für die Bestimmung der Reaktionsenergien im Schritt g) Boltzmann gewichtete Mittelwerte der Anregungsenergien, Absorptionsstärken, sowie Gesamtenergien verwendet werden, wobei die Boltzmann-Gewichte auf

dichtefunktionaltheoretischem Niveau, besonders bevorzugt auf Basis von DFT(BP86/TZVP) Geometrieoptimierungen mit anschließenden DFT(BH-LYP/TZVP) Einzelpunktrechnungen jeweils unter Verwendung von COSMO, berechnet werden.

Bei einer weiteren bevorzugten Ausführungsform des erfindungsgemäßen Verfahrens kann im Schritt e) die zeitabhängige DFT(BH-LYP/TZVP) Methode zur Berechnung der Absorptionsspektren verwendet und der systematische Fehler mit +0.7 Elektronenvolt angenommen werden.

Bevorzugt ist auch, wenn im Schritt e) die zeitabhängige DFT(BH-LYP/TZVP) Methode in Verbindung mit dem COSMO Modell verwendet wird und der systematische Fehler mit +0.56 Elektronenvolt angenommen wird.

Die Photopolymer-Formulierung kann bevorzugt Matrixpolymere, weiter bevorzugt vernetzte Matrixpolymere und besonders bevorzugt dreidimensional vernetzte Matrixpolymere enthalten. Ganz besonders bevorzugt können die dreidimensional vernetzte Matrixpolymere Polyurethane sein. Diese Polyurethane können durch Umsetzung wenigstens einer Polyisocyanat-Komponente a) und wenigstens einer isocyanatreaktiven Komponente b) hergestellt werden.

Die Polyisocyanat-Komponente a) umfasst bevorzugt wenigstens eine organische Verbindung, die bevorzugt wenigstens zwei NCO-Gruppen aufweist (Polyisocyanat).

Als Polyisocyanat können alle dem Fachmann an sich gut bekannten Verbindungen oder deren Mischungen eingesetzt werden. Diese Verbindungen können auf aromatischer, araliphatischer, aliphatischer oder cycloaliphatischer Basis sein. In untergeordneten Mengen kann die Polyisocyanat-Komponente a) auch Monoisocyanate, d.h. organische Verbindungen mit einer NCO-Gruppe, und / oder ungesättigte Gruppen enthaltende Polyisocyanate umfassen.

Beispiele für geeignete Polyisocyanate sind Butylendiisocyanat, Hexamethylendiisocyanat (HDI), 2,2,4-Trimethylhexamethylendiisocyanat und dessen Isomere (TMDI), Isophorondiisocyanat (IPDI), 1,8-Diisocyanato-4-(isocyanatomethyl)-octan, die isomeren Bis-(4,4'-isocyanatocyclohexyl)methane und deren Mischungen beliebigen Isomerengehalts, Isocyanatomethyl-1,8-octandiisocyanat, 1,4-Cyclohexylendiisocyanat, die isomeren Cyclohexandimethylendiisocyanate, 1,4-Phenylendiisocyanat, 2,4- und / oder 2,6-Toluylendiisocyanat, 1,5-Naphthylendiisocyanat, 2,4'- und / oder 4,4'-Diphenylmethandiisocyanat, Triphenylmethan-4,4',4''-triisocyanat oder beliebige Mischungen der vorgenannten Verbindungen.

Monomere Di- oder Triisocyanate mit Urethan-, Harnstoff-, Carbodiimid-, Acylharnstoff-, Isocyanurat-, Allophanat-, Biuret-, Oxadiazintrion-, Uretdion- und/oder Iminooxadiazindionstrukturen können ebenfalls verwendet werden.

Bevorzugt sind Polyisocyanate auf Basis aliphatischer und/oder cycloaliphatischer Di-oder Triisocyanate.

Besonders bevorzugt handelt es sich bei den Polyisocyanaten um di- oder oligomerisierte aliphatische und / oder cycloaliphatische Di- oder Triisocyanate.

Ganz besonders bevorzugte Polyisocyanate sind Isocyanurate, Uretdione und / oder Iminooxadiazindione basierend auf HDI, TMDI, 1,8-Diisocyanato-4-(isocyanatomethyl)-octan oder deren Mischungen.

Die Polyisocyanat-Komponente a) kann auch NCO-funktionelle Prepolymere umfassen oder daraus bestehen. Die Prepolymere können Urethan-, Allophanat-, Biuret- und / oder Amidgruppen aufweisen. Derartige Prepolymere sind beispielsweise durch Umsetzung von Polyisocyanaten a1) mit isocyanatreaktiven Verbindungen a2) erhältlich.

Als Polyisocyanate a1) eignen sich alle bekannten aliphatischen, cycloaliphatischen, aromatischen oder araliphatischen Di- und Triisocyanate. Daneben können auch die bekannten höher-molekularen Folgeprodukte monomerer Di- und / oder Triisocyanate mit Urethan-, Harnstoff-, Carbodiimid-, Acylharnstoff-, Isocyanurat-, Allophanat-, Biuret-, Oxadiazintrion-, Uretdion-, Iminooxadiazindionstruktur jeweils einzeln oder in beliebigen Mischungen untereinander eingesetzt werden.

Beispiele für geeignete monomere Di- oder Triisocyanate, die als Polyisocyanat a1) eingesetzt werden können, sind Butylendiisocyanat, Hexamethylendiisocyanat, Isophorondiisocyanat, Trimethyl-hexamethylen-diisocyanat (TMDI), 1,8-Diisocyanato-4-(isocyanatomethyl)-octan, Isocyanatomethyl-1,8-octandiisocyanat (TIN), 2,4- und / oder 2,6-Toluendiisocyanat.

Als isocyanatreaktive Verbindungen a2) können bevorzugt OH-funktionelle Verbindungen verwendet werden. Dabei kann es sich insbesondere um Polyole handeln. Ganz besonders bevorzugt können als isocyanatreaktive Verbindung a2) die weiter unten beschriebenen Polyole der Komponente b) verwendet werden.

Es ist ebenfalls möglich, als isocyanatreaktive Verbindungen a2) Amine einzusetzen. Beispiele geeigneter Amine sind Ethylendiamin, Diethylentriamin, Triethylentetramin, Propylendiamin, Diaminocyclohexan, Diaminobenzol, Diaminobisphenyl, difunktionelle Polyamine wie z.B. die Jeffamine®, aminterminierte Polymere, insbesondere mit zahlenmittleren Molmassen bis 10000 g/Mol. Mischungen der vorgenannten Amine können ebenfalls verwendet werden.

Bevorzugt ist auch, wenn die isocyanatreaktiven Verbindungen a2) eine zahlenmittlere Molmasse von $\geq$ 200 und $\leq$ 10000 g/Mol, weiter bevorzugt $\geq$ 500 und $\leq$ 8500 g/Mol und ganz besonders bevorzugt $\geq$ 1000 und $\leq$ 8200 g/Mol aufweisen.

Die Prepolymere der Polyisocyanat-Komponente a) können insbesondere einen Restgehalt an freiem monomerem Isocyanat < 1 Gew.-%, besonders bevorzugt <0.5 Gew.-% und ganz besonders bevorzugt < 0.2 Gew.-% aufweisen.

Die Polyisocyanat-Komponente a) kann auch Mischungen der vorgenannten Polyisocyanate und Prepolymere

umfassen.

Es ist gegebenenfalls auch möglich, dass die Polyisocyanat-Komponente a) anteilsmäßig Polyisocyanate enthält, die teilweise mit isocyanat-reaktiven, ethylenisch ungesättigten Verbindungen umgesetzt sind. Bevorzugt werden hierbei als isocyanat-reaktive, ethylenisch ungesättigte Verbindungen $\alpha,\beta$-ungesättigte Carbonsäurederivate wie Acrylate, Methacrylate, Maleinate, Fumarate, Maleimide, Acrylamide, sowie Vinylether, Propenylether, Allylether und Dicyclopentadienyl-Einheiten enthaltende Verbindungen, die mindestens eine gegenüber Isocyanaten reaktive Gruppe aufweisen, eingesetzt. Besonders bevorzugt sind Acrylate und Methacrylate mit mindestens einer isocyanatreaktiven Gruppe.

Der Anteil der Polyisocyanate in der Polyisocyanat-Komponente a), der teilweise mit isocyanat-reaktiven, ethylenisch ungesättigten Verbindungen umgesetzt ist, kann 0 bis 99 Gew.-%, bevorzugt 0 bis 50 Gew.-%, besonders bevorzugt 0 bis 25 Gew.-% und ganz besonders bevorzugt 0 bis 15 Gew.-% betragen.

Es ist gegebenenfalls auch möglich, dass die Polyisocyanat-Komponente a) vollständig oder anteilsmäßig Polyisocyanate enthält, die ganz oder teilweise mit aus der Beschichtungstechnologie bekannten Blockierungsmitteln umgesetzt sind. Beispiel für Blockierungsmittel sind Alkohole, Lactame, Oxime, Malonester, Alkylacetoacetate, Triazole, Phenole, Imidazole, Pyrazole sowie Amine, wie z.B. Butanonoxim, Diisopropylamin, 1,2,4-Triazol, Dimethyl-1,2,4-triazol, Imidazol, Malonsäurediethylester, Acetessigester, Acetonoxim, 3,5-Dimethylpyrazol, $\varepsilon$-Caprolactam, N-tert.-Butyl-benzylamin, Cyclopentanoncarboxyethylester oder deren Mischungen.

Besonders bevorzugt ist, wenn die Polyisocyanat-Komponente a) ein aliphatisches Polyisocyanat oder ein aliphatisches Prepolymer und bevorzugt ein aliphatisches Polyisocyanat oder aliphatisches Prepolymer mit primären NCO-Gruppen umfasst oder daraus besteht.

Die isocyanatreaktive Komponente b) umfasst bevorzugt wenigstens eine organische Verbindung, die wenigstens zwei isocyanatreaktive Gruppen aufweist (isocyanatreaktive Verbindung). Im Rahmen der vorliegenden Erfindung werden als isocyanatreaktive Gruppen Hydroxy-, Amino- oder Thiogruppen angesehen.

Als isocyanatreaktive Komponente können alle Systeme eingesetzt werden, die im Mittel wenigstens 1.5 und bevorzugt 2 bis 3 isocyanatreaktive Gruppen aufweisen.

Isocyanatreaktive Gruppen im Rahmen der vorliegenden Erfindung sind bevorzugt Hydroxy-, Amino- oder Thiogruppen, besonders bevorzugt sind Hydroxyverbindungen.

Geeignete polyfunktionelle, isocyanatreaktive Verbindungen sind beispielsweise Polyester-, Polyether-, Polycarbonat-, Poly(meth)acrylat- und/oder Polyurethanpolyole.

Als Polyesterpolyole sind beispielsweise lineare Polyesterdiole oder verzweigte Polyester-polyole geeignet, wie sie in bekannter Weise aus aliphatischen, cycloaliphatischen oder aromatischen Di- bzw. Polycarbonsäuren bzw. ihren Anhydriden mit mehrwertigen Alkoholen einer OH-Funktionalität $\geq 2$ erhalten werden.

Beispiele für solche Di- bzw. Polycarbonsäuren bzw. Anhydride sind Bernstein-, Glutar-, Adipin-, Pimelin-, Kork-, Azelain-, Sebacin-, Nonandicarbon-, Decandicarbon-, Terephthal-, Isophthal-, o-Phthal-, Tetrahydrophthal-, Hexahydrophthal- oder Trimellitsäure sowie Säureanhydride wie o-Phthal-, Trimellit- oder Bernsteinsäureanhydrid oder deren beliebige Gemische untereinander.

Beispiele für geeignete Alkohole sind Ethandiol, Di-, Tri-, Tetraethylenglykol, 1,2-Propandiol, Di-, Tri-, Tetrapropylenglykol, 1,3-Propandiol, Butandiol-1,4, Butandiol-1,3, Butandiol-2,3, Pentandiol-1,5, Hexandiol-1,6, 2,2-Dimethyl-1,3-propandiol, 1,4-Dihydroxy-cyclohexan, 1,4-Dimethylolcyclohexan, Octandiol-1,8, Decandiol-1,10, Dodecandiol-1,12, Trimethylolpropan, Glycerin oder deren beliebige Gemische untereinander.

Die Polyesterpolyole können auch auf natürlichen Rohstoffen wie Rizinusöl basieren. Ebenfalls möglich ist, dass die Polyesterpolyole auf Homo- oder Mischpolymerisaten von Lactonen basieren, wie sie bevorzugt durch Anlagerung von Lactonen bzw. Lactongemischen wie Butyrolacton, $\varepsilon$-Caprolacton und/oder Methyl-$\varepsilon$-caprolacton an hydroxyfunktionelle Verbindungen wie mehrwertige Alkohole einer OH-Funktionalität $\geq 2$ beispielsweise der vorstehend genannten Art erhalten werden können.

Solche Polyesterpolyole haben bevorzugt zahlenmittlere Molmassen von 400 bis 4000 g/Mol, besonders bevorzugt von 500 bis 2000 g/Mol. Ihre OH-Funktionalität beträgt bevorzugt 1.5 bis 3.5, besonders bevorzugt 1.8 bis 3.0.

Geeignete Polycarbonatpolyole sind in an sich bekannter Weise durch Umsetzung von organischen Carbonaten oder Phosgen mit Diolen oder Diol-Mischungen zugänglich.

Geeignete organische Carbonate sind Dimethyl-, Diethyl- und Diphenylcarbonat.

Geeignete Diole bzw. Mischungen umfassen die an sich im Rahmen der Polyestersegmente genannten mehrwertigen Alkoholen einer OH-Funktionalität $\geq 2$, bevorzugt Butandiol-1,4, Hexandiol-1,6 und/oder 3-Methylpentandiol, oder auch Polyesterpolyole können zu Polycarbonatpolyolen umgearbeitet werden.

Solche Polycarbonatpolyole haben bevorzugt zahlenmittlere Molmassen von 400 bis 4000 g/Mol, besonders bevorzugt von 500 bis 2000 g/Mol. Die OH-Funktionalität dieser Polyole beträgt bevorzugt 1.8 bis 3.2, besonders bevorzugt 1.9 bis 3.0.

Geeignete Polyetherpolyole sind gegebenenfalls blockweise aufgebaute Polyadditions-produkte cyclischer Ether an OH- oder NH-funktionelle Startermoleküle.

Geeignete cyclische Ether sind beispielsweise Styroloxide, Ethylenoxid, Propylenoxid, Tetrahydrofuran, Butylenoxid, Epichlorhydrin, sowie ihre beliebigen Mischungen.

Als Starter können die an sich im Rahmen der Polyesterpolyole genannten mehrwertigen Alkohole einer OH-Funktionalität ≥ 2 sowie primäre oder sekundäre Amine und Amino-alkohole verwendet werden.

Bevorzugte Polyetherpolyole sind solche der vorgenannten Art ausschließlich basierend auf Propylenoxid oder statistische oder Block-Copolymere basierend auf Propylenoxid mit weiteren 1-Alkylenoxiden, wobei der 1-Alykenoxidanteil nicht höher als 80 Gew.-% ist. Besonders bevorzugt sind Propylenoxidhomopolymere sowie statistische oder Block-Copolymere, die Oxyethylen-, Oxypropylen- und/oder Oxybutyleneinheiten aufweisen, wobei der Anteil der Oxypropyleneinheiten bezogen auf die Gesamtmenge aller Oxyethylen-, Oxypropylen- und Oxybutyleneinheiten mindestens 20 Gew.-%, bevorzugt mindestens 45 Gew.-% ausmacht. Oxypropylen- und Oxybutylen umfasst hierbei alle jeweiligen linearen und verzweigten $C_3$- und $C_4$-Isomere.

Solche Polyetherpolyole haben bevorzugt zahlenmittlere Molmassen von 250 bis 10000 g/Mol, besonders bevorzugt von 500 bis 8500 g/Mol und ganz besonders bevorzugt von 600 bis 4500 g/Mol. Die OH-Funktionalität beträgt bevorzugt 1.5 bis 4.0, besonders bevorzugt 1.8 bis 3.1.

Daneben sind als Bestandteile der Polyol-Komponente b) als polyfunktionelle, isocyanatreaktive Verbindungen auch niedermolekulare, d.h. mit Molekulargewichten kleiner 500 g/mol, kurzkettige, d.h. 2 bis 20 Kohlenstoffatome enthaltende aliphatische, araliphatische oder cycloaliphatische di, tri oder polyfunktionelle Alkohole geeignet.

Dies können beispielsweise sein Ethylenglykol, Diethylenglykol, Triethylenglykol, Tetraethylenglykol, Dipropylenglykol, Tripropylenglykol, 1,2-Propandiol, 1,3-Propandiol, 1,4-Butandiol, Neopentylglykol, 2-Ethyl-2-butylpropandiol, Trimethylpentandiol, stellungs-isomere Diethyloctandiole, 1,3-Butylenglykol, Cyclohexandiol, 1,4-Cyclohexandimethanol, 1,6-Hexandiol, 1,2- und 1,4-Cyclohexandiol, hydriertes Bisphenol A, (2,2-Bis(4-hydroxy-cyclohexyl)propan), 2,2-Dimethyl-3-hydroxypropionsäure (2,2-dimethyl-3-hydroxypropyl-ester). Beispiele geeigneter Triole sind Trimethylolethan, Trimethylolpropan oder Glycerin. Geeignete höherfunktionelle Alkohole sind Ditrimethylolpropan, Pentaerythrit, Dipenta-erythrit oder Sorbit.

Besonders bevorzugt ist, wenn die Polyolkomponente ein difunktioneller Polyether-, Polyester oder ein Polyether-polyester-block-copolyester oder ein Polyether-Polyester-Blockcopolymer mit primären OH-Funktionen ist. Die Photopolymer-Formulierung kann bevorzugt auch ein Schreibmonomer umfassen.

Das Schreibmonomer kann wenigstens ein mono- und / oder ein multifunktionales Schreibmonomer umfassen, wobei es sich insbesondere um mono- und multifunktionelle Acrylat-Schreibmonomere handeln kann. Besonders bevorzugt kann das Schreibmonomer wenigstens ein monofunktionelles und ein multifunktionelles Urethan(meth)acrylat umfassen.

Bei den Acrylat-Schreibmonomeren kann es sich insbesondere um Verbindungen der allgemeinen Formel (II)

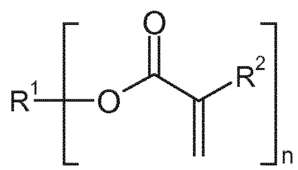
(II)

handeln, bei denen n≥1 und n≤4 ist und $R^1$ ein linearer, verzweigter, cyclischer oder heterocyclischer unsubstituierter oder gegebenenfalls auch mit Heteroatomen substituierter organischer Rest und/oder $R^2$ Wasserstoff, ein linearer, verzweigter, cyclischer oder heterocyclischer unsubstituierter oder gegebenenfalls auch mit Heteroatomen substituierter organischer Rest ist. Besonders bevorzugt ist $R^2$ Wasserstoff oder Methyl und/oder $R^1$ ein linearer, verzweigter, cyclischer oder heterocyclischer unsubstituierter oder gegebenenfalls auch mit Heteroatomen substituierter organischer Rest.

Ebenso ist es möglich, dass weitere ungesättigte Verbindungen wie $\alpha,\beta$-ungesättigte Carbonsäurederivate wie Acrylate, Methacrylate, Maleinate, Fumarate, Maleimide, Acrylamide, weiterhin Vinylether, Propenylether, Allylether und Dicyclopentadienyl-Einheiten enthaltende Verbindungen sowie olefinisch ungesättigte Verbindungen wie z.B. Styrol, $\alpha$-Methylstyrol, Vinyltoluol, Olefinine, wie z.B. 1-Octen und/oder 1-Decen, Vinylestern, (Meth)acrylnitril, (Meth)acrylamid, Methacrylsäure, Acrylsäure zugesetzt werden. Bevorzugt sind aber Acrylate und Methacrylate.

Als Acrylate bzw. Methacrylate werden allgemein Ester der Acrylsäure bzw. Methacrylsäure bezeichnet. Beispiele verwendbarer Acrylate und Methacrylate sind Methylacrylat, Methylmethacrylat, Ethylacrylat, Ethylmethacrylat, Ethoxyethylacrylat, Ethoxyethylmethacrylat, n-Butylacrylat, n-Butylmethacrylat, tert.-Butylacrylat, tert.-Butylmethacrylat, Hexylacrylat, Hexylmethacrylat, 2-Ethylhexylacrylat, 2-Ethylhexylmethacrylat, Butoxyethylacrylat, Butoxyethylmethacrylat, Laurylacrylat, Laurylmethacrylat, Isobornylacrylat, Isobornylmethacrylat, Phenylacrylat, Phenylmethacrylat, p-Chlorphenylacrylat, p-Chlorphenylmethacrylat, p-Bromphenylacrylat, p-Bromphenyl-methacrylat, 2,4,6-Trichlorphenylacrylat, 2,4,6-Trichlorphenylmethacrylat, 2,4,6-Tribromphenyl-acrylat, 2,4,6-Tribromphenylmethacrylat, Pentachlorphenylacrylat, Pentachlorphenylmethacrylat, Pentabromphenylacrylat, Pentabromphenylmethacrylat, Pentabrombenzylacrylat, Pentabrombenzylmethacrylat, Phenoxyethylacrylat, Phenoxyethylmethacrylat, Phenoxyethoxyethylacrylat, Phenoxyethoxyethylmethacrylat, Phenylthioethylacrylat, Phenylthioethylmethacrylat, 2-Naphthylacrylat, 2-Naphthylmethacrylat, 1,4-Bis-(2-thionaphthyl)-2-butylacrylat, 1,4-Bis-(2-thionaphthyl)-2-butylmethacrylat, Propan-2,2-diylbis[(2,6-dibrom-4,1-phenylen)oxy(2-{[3,3,3-tris(4-chlorphenyl)-propanoyl]-oxy}propan-3,1-diyl)oxyethan-2,1-diyl]-diacrylat, Bisphenol A Diacrylat, Bisphenol A Dimethacrylat, Tetrabromobisphenol A Diacrylat, Tetrabromobisphenol A Dimethaciylat sowie deren ethoxylierte Analogverbindungen, N-Carbazolylacrylate, um nur eine Auswahl verwendbarer Acrylate und Methacrylate zu nennen.

Selbstverständlich können als Acrylate auch Urethanacrylate verwendet werden. Unter Urethanacrylaten versteht man Verbindungen mit mindestens einer Acrylsäureestergruppe, die zusätzlich über mindestens eine Urethanbindung verfügen. Es ist bekannt, dass solche Verbindungen durch Umsetzung eines Hydroxy-funktionellen Acrylsäureesters mit einer Isocyanat-funktionellen Verbindung erhalten werden können.

Beispiele hierfür verwendbarer Isocyanat-funktionelle Verbindungen sind aromatische, araliphatische, aliphatische und cycloaliphatische Di-, Tri- oder Polyisocyanate. Es können auch Mischungen solcher Di-, Tri- oder Polyisocyanate eingesetzt werden. Beispiele geeigneter Di-, Tri- oder Polyisocyanate sind Butylendiisocyanat, Hexamethylendiisocyanat (HDI), Isophorondiisocyanat (IPDI), 1,8-Diisocyanato-4-(isocyanatomethyl)octan, 2,2,4- und/oder 2,4,4-Trimethylhexamethylendiisocyanat, die isomeren Bis(4,4'-isocyanatocyclohexyl)methane und deren Mischungen beliebigen Isomerengehalts, Isocyanatomethyl-1,8-octandiisocyanat, 1,4-Cyclohexylendiisocyanat, die isomeren Cyclohexandimethylendiisocyanate, 1,4-Phenylendiisocyanat, 2,4- und/oder 2,6-Toluylendiisocyanat, 1,5-Naphthylendiisocyanat, 2,4'- oder 4,4'-Diphenylmethandiisocyanat, 1,5-Naphthylendiisocyanat, m-Methylthiophenylisocyanat, Triphenylmethan-4,4',4''-triisocyanat und Tris(p-isocyanatophenyl)thiophosphat oder deren Derivate mit Urethan-, Harnstoff-, Carbodiimid-, Acylharnstoff-, Isocyanurat-, Allophanat-, Biuret-, Oxadiazintrion-, Uretdion-, Iminooxadiazindionstruktur und Mischungen derselben. Bevorzugt sind dabei aromatische oder araliphatische Di-, Tri- oder Polyisocyanate.

Als hydroxyfunktionelle Acrylate oder Methacrylate für die Herstellung von Urethanacrylaten kommen beispielsweise Verbindungen in Betracht wie 2-Hydroxyethyl(meth)acrylat, Polyethylenoxid-mono-(meth)acrylate, Polypropylenoxidmono(meth)acrylate, Polyalkylenoxidmono(meth)-acrylate, Poly(ε-caprolacton)mono(meth)acrylate, wie z.B. Tone® M100 (Dow, Schwalbach, DE), 2-Hydroxypropyl-(meth)acrylat, 4-Hydroxybutyl(meth)acrylat, 3-Hydroxy-2,2-dimethylpropyl-(meth)acrylat, Hydroxypropyl(meth)acrylat, Acrylsäure-(2-hydroxy-3-phenoxypropylester), die hydroxyfunktionellen Mono-, Di-oder Tetraacrylate mehrwertiger Alkohole wie Trimethylolpropan, Glycerin, Pentaerythrit, Dipentaerythrit, ethoxyliertes, propoxyliertes oder alkoxyliertes Trimethylolpropan, Glycerin, Pentaerythrit, Dipentaerythrit oder deren technische Gemische. Bevorzugt sind 2-Hydroxyethylacrylat, Hydroxypropylacrylat, 4-Hydroxybutylacrylat und Poly(ε-caprolacton)mono(meth)acrylate. Darüber hinaus sind als isocyanat-reaktive oligomere oder polymere ungesättigte Acrylat- und/oder Methacrylatgruppen enthaltende Verbindungen alleine oder in Kombination mit den vorgenannten monomeren Verbindungen geeignet. Ebenfalls verwendet werden können die an sich bekannten hydroxylgruppenhaltigen Epoxy(meth)acrylate mit OH-Gehalten von 20 bis 300 mg KOH/g oder hydroxylgruppenhaltige Polyurethan(meth)acrylate mit OH-Gehalten von 20 bis 300 mg KOH/g oder acrylierte Polyacrylate mit OH-Gehalten von 20 bis 300 mg KOH/g sowie deren Mischungen untereinander und Mischungen mit hydroxylgruppenhaltigen ungesättigten Polyestern sowie Mischungen mit Polyester(meth)acrylaten oder Mischungen hydroxylgruppenhaltiger ungesättigter Polyester mit Polyester(meth)acrylaten.

Bevorzugt sind insbesondere Urethanacrylate erhältlich aus der Umsetzung von Tris(p-isocyanatophenyl)thiophosphat und m-Methylthiophenylisocyanat mit alkoholfunktionellen Acrylaten wie Hydroxyethyl(meth)acrylat, Hydroxypropyl(meth)acrylat und Hydroxybutyl(meth)acrylat.

Gemäß einer weiteren bevorzugten Ausführungsform ist vorgesehen, dass die Photopolymer-Formulierung zusätzlich Urethane als Weichmacher enthält, wobei die Urethane insbesondere mit wenigstens einem Fluoratom substituiert sein können.

Bevorzugt können die Urethane die allgemeine Formel (III)

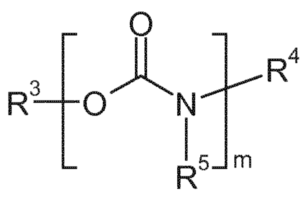

(III)

haben, in der $m \geq 1$ und $m \leq 8$ ist und $R^3$ ein linearer, verzweigter, cyclischer oder heterocyclischer unsubstituierter oder gegebenenfalls auch mit Heteroatomen substituierter organischer Rest und/oder $R^4$, $R^5$ unabhängig voneinander Wasserstoff sind, wobei bevorzugt mindestens einer der Reste $R^3$, $R^4$, $R^5$ mit wenigstens einem Fluoratom substituiert ist und besonders bevorzugt $R^3$ ein organischer Rest mit mindestens einem Fluoratom ist. Besonders bevorzugt ist $R^5$ ein

linearer, verzweigter, cyclischer oder heterocyclischer unsubstituierter oder gegebenenfalls auch mit Heteroatomen wie beispielsweise Fluor substituierter organischer Rest.

**Beispiele:**

Die Erfindung wird an Hand der folgenden Beispiele näher erläutert. Dabei wird zunächst die Synthese und Charakterisierung der Beispielmoleküle beschrieben und im Anschluss deren Behandlung im Rahmen des erfindungsgemäßen Verfahrens.

**Substanzen:**

Die verwendeten Farbstoffe, Salze sowie Lösungsmittel und Reagenzien wurden, soweit nicht unten ihre Herstellung beschrieben ist, im Chemikalienhandel bezogen.

| | |
|---|---|
| CGI-909 | Tetrabutylammonium-tris(3-chlor-4-methylphenyl)(hexyl)borat, [1147315-11-4] ist ein von der BASF SE, Basel, Schweiz, hergestelltes Produkt. |
| Desmorapid® Z | Dibutylzinn-dilaurat [77-58-7], Produkt der Bayer MaterialScience AG, Leverkusen, Deutschland. |
| Desmodur® N 3900 | Produkt der Bayer MaterialScience AG, Leverkusen, DE, Hexandiisocyanatbasiertes Polyisocyanat, Anteil an Iminooxadiazindion mindestens 30 %, NCO-Gehalt: 23.5 %. |
| Fomrez® UL 28 | Urethanisierungskatalysator, Handelsprodukt der Momentive Performance Chemicals, Wilton, CT, USA. |
| Irgacure® 250 | (4-Methylphenyl)-[4-(2-methylpropyl)phenyl]iodonium-hexafluorophosphat, [344562-80-7], 75%-ige Lösung in Propylencarbonat, ist ein von der BASF SE, Basel, Schweiz, hergestelltes Produkt |
| EDB | Ethyl-(4-dimethylamino)benzoat [10287-53-3], Sigma Aldrich |
| New Methylene Blue | C. I. Basic Blue 24, [als Chlorid: 1934-16-3], Sigma Aldrich |

**Herstellung der Komponenten**

**Herstellung von Polyol 1:**

In einem 1 L Kolben wurden 0.18 g Zinnoctoat, 374.8 g ε-Caprolacton und 374.8 g eines difunktionellen Polytetrahydrofuranpolyetherpolyols (Equivalentgewicht 500 g/Mol OH) vorgelegt und auf 120 °C aufgeheizt und so lange auf dieser Temperatur gehalten, bis der Festgehalt (Anteil der nicht-flüchtigen Bestandteile) bei 99.5 Gew.-% oder darüber lag. Anschließend wurde abgekühlt und das Produkt als wachsiger Feststoff erhalten.

**Herstellung des Acrylates 1 (Phosphorthioyltris(oxy-4,1-phenyleniminocarbonyloxyethan-2,1-diyl)-triacrylat):**

In einem 500 mL Rundkolben wurden 0.1 g 2,6-Di-tert.-butyl-4-methylphenol, 0.05 g Dibutylzinn-dilaurat (Desmorapid® Z, Bayer MaterialScience AG, Leverkusen, Deutschland) sowie und 213.07 g einer 27 %-igen Lösung von Tris(p-isocyanatophenyl)thiophosphat in Ethylacetat (Desmodur® RFE, Produkt der Bayer MaterialScience AG, Leverkusen, Deutschland) vorgelegt und auf 60 °C erwärmt. Anschließend wurden 42.37 g 2-Hydroxyethylacrylat zugetropft und die Mischung weiter auf 60 °C gehalten, bis der Isocyanatgehalt unter 0.1 % gesunken war. Danach wurde abgekühlt und im Vakuum das Ethylacetat vollständig entfernt. Das Produkt wurde als teilkristalliner Feststoff erhalten.

**Herstellung des Acrylates 2 2-({[3-(Methylsulfanyl)phenyl]carbamoyl}oxy)ethylprop-2-enoat):**

In einem 100 mL Rundkolben wurden 0.02 g 2,6-Di-tert.-butyl-4-methylphenol, 0.01 g Desmorapid® Z, 11.7 g 3-(Methylthio)phenylisocyanat vorgelegt und auf 60 °C erwärmt. Anschließend wurden 8.2 g 2-Hydroxyethylacrylat zugetropft und die Mischung weiter auf 60 °C gehalten, bis der Isocyanatgehalt unter 0.1 % gesunken war. Danach wurde abgekühlt. Das Produkt wurde als hellgelbe Flüssigkeit erhalten.

**Herstellung des Additives 1: (Bis(2,2,3,3,4,4,5,5,6,6,7,7-dodecafluorheptyl)-(2,2,4-trimethylhexan-1,6-diyl)biscarbamat):**

In einem Rundkolben wurden 0.02 g Desmorapid Z und 3.6 g 2,4,4-Trimethylhexane-1,6-diisocyanat vorgelegt und auf 70 °C erwärmt. Anschließend wurden 11.39 g 2,2,3,3,4,4,5,5,6,6,7,7-Dodecafluorheptan-1-ol zugetropft und die Mischung weiter auf 70 °C gehalten, bis der Isocyanatgehalt unter 0.1 % gesunken war. Danach wurde abgekühlt. Das Produkt wurde als farbloses Öl erhalten.

**Synthese des Farbstoffs FI: Pyridinsalz von 5-[5-(Hexahydro-1,3-diethyl-4,6-dioxo-2-thioxo-5-pyrimidinyl)-2,4-propenylidene]-1,3-diethyl-2-thio-barbitursäure**

4.00 g 1,3-Diethyl-2-thiobarbitursäure und 1.64 g 1,1,3,3-Tetramethoxypropan wurden in 10 mL Pyridin 10 h bei 90 °C gerührt. Nach dem Abkühlen wurde mit 30 mL Toluol verdünnt, abgesaugt, mit 3x10 mL Toluol und schließlich 50 mL Wasser gewaschen und bei 50 °C im Vakuum getrocknet. Man erhielt 2.33 g (45.3 % d. Th.) eines roten Kristallisats der Formel

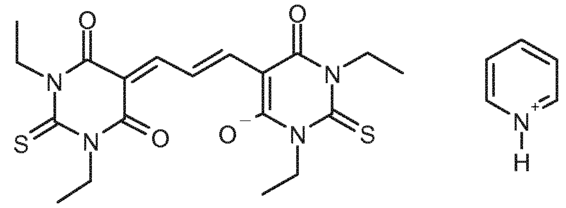

.

## Herstellung der Medien zur Bestimmung der holographischen Eigenschaften

**Beispiel Medium 1**

3.38 g der Polyol-Komponente 1 wurden mit 2.00 g Acrylat 1, 2.00 g Acrylat 2, 1.50 g Additiv 1, 0.10 g CGI 909 (Produkt der Fa. BASF SE, Basel, Schweiz), 0.010 g New Methylene Blue (= F7) und 0.35 g N-Ethylpyrrolidon bei 60 °C gemischt, so dass eine klare Lösung erhalten wurde. Anschließend wurde auf 30 °C abgekühlt, 0.65 g Desmodur® N3900 (Handelsprodukt der Bayer MaterialScience AG, Leverkusen, DE, Hexandiisocyanat-basiertes Polyisocyanat, Anteil an Iminooxadiazindion mindestens 30 %, NCO-Gehalt: 23.5 %) zugegeben und erneut gemischt. Schließlich wurden 0.01 g Fomrez UL 28 (Urethanisierungskatalysator, Handelsprodukt der Fa. Momentive Performance Chemicals, Wilton, CT, USA) zugegeben und erneut kurz gemischt. Die erhaltene, flüssige Masse wurde dann auf eine Glasplatte gegeben und dort mit einer zweiten Glasplatte abgedeckt. Dieser Probenkörper wurde 12 Stunden bei Raumtemperatur liegen gelassen und gehärtet.

**Beispiel Medium 2**

3.38 g der Polyol-Komponente 1 wurden mit 2.00 g Acrylat 1, 2.00 g Acrylat 2, 1.50 g Additiv 1, 0.10 g Irgacure® 250 (Produkt der Fa. BASF SE, Basel, Schweiz), 0.10 g EDB, 0.010 g des Farbstoffs F1 und 0.35 g N-Ethylpyrrolidon bei 60 °C gemischt, so dass eine klare Lösung erhalten wurde. Anschließend wurde auf 30 °C abgekühlt, 0.65 g Desmodur® N3900 (Handelsprodukt der Bayer MaterialScience AG, Leverkusen, DE, Hexandiisocyanat-basiertes Polyisocyanat, Anteil an Iminooxadiazindion mindestens 30 %, NCO-Gehalt: 23,5 %) zugegeben und erneut gemischt. Schließlich wurden 0.01 g Fomrez UL 28 (Urethanisierungskatalysator, Handelsprodukt der Fa. Momentive Performance Chemicals, Wilton, CT, USA) zugegeben und erneut kurz gemischt. Die erhaltene, flüssige Masse wurde dann auf eine Glasplatte gegeben und dort mit einer zweiten Glasplatte abgedeckt. Dieser Probenkörper wurde 12 Stunden bei Raumtemperatur liegen gelassen und gehärtet.

## Holographische Belichtung der Photopolymerformulierungen:

In das Photopolymer wurde ein Hologramm mittels einer Messanordnung gemäß Figur 1 einbelichtet. Es handelte sich um monochromatische Hologramme mit 633 nm oder 532 nm Laserwellenlänge. Dazu wurden die wie oben beschrieben hergestellten Beispiel-Medien mittels eines Probenhalters in die Messanordnung eingebracht.

Der Strahl eines Lasers (Emissionswellenlänge 633 nm oder 532 nm) wurde mit Hilfe einer optionalen Aufweitungslinse (AF) und der Kollimationslinse (CL), die nach dem Shutter S platziert ist, auf einen Durchmesser von ~ 3 - 4

cm aufgeweitet. Der Durchmesser des aufgeweiteten Laserstrahls wurde dabei durch die Apertur des geöffneten Shutters bestimmt. Es wurde bewusst auf eine inhomogene Intensitätsverteilung des aufgeweiteten Laserstrahles geachtet. So betrug die Randintensität PR ~ nur die Hälfte der Intensität PZ im Zentrum des aufgeweiteten Laserstrahls. P war hier als Leistung/ Fläche zu verstehen. Der aufgeweitete Laserstrahl durchlief zunächst eine schräg zum Strahl gestellte Glasplatte, die als Shearing Plate (SP) diente. Anhand des nach oben gespiegelten Interferenzmusters, das von den zwei Glasoberflächenreflexen der SP erzeugt wurde, konnte erkannt werden, ob der Laser stabil im Single Mode emittiert. Dann war auf einer über der SP platziert Mattscheibe ein Muster aus dunklen und hellen Streifen zu sehen. Nur wenn Single Mode Emission gegeben war, wurden holographischen Belichtungen durchgeführt. Im Falle der DPSS Laser konnte der Single Mode durch einstellen des Pumpstroms erreicht werden. Der aufgeweitete Strahl durchlief das etwa 15° schräg gestellten Photopolymer (P), dieser Teil bildete den Referenzstrahl, um dann vom zu P parallel angeordneten Objekt (O) wieder zurück in P reflektiert zu werden. Dieser Teil bildete dann den Signalstrahl der Denisyuk-Anordnung.

Die Interferenz von Signal- und Referenzstrahl in P erzeugte das Hologramm im Photopolymer. O bestand aus einer mit weißem Papier bedeckten Metallplatte, wobei die Papierseite P zugewandt war. Auf dem Papier befand sich ein quadratisches Raster erzeugt durch schwarze Linien. Die Kantenlänge eines Quadrates betrug 0.5 cm. Dieses Raster wurde bei der holographischen Belichtung von P mit im Hologramm abgebildet.

Die mittlere Belichtungsdosis $E_{ave}$ wurde durch die Öffnungszeit t von S eingestellt. Bei fixierter Laserleistung I stellte t daher die zu $E_{ave}$ proportionale Größe dar. Da der aufgeweitete Laserstrahl eine inhomogenene (glockenförmige) Intensitätsverteilung besaß, variiert die lokale Dosis E zur Erzeugung des Hologramms in P. Dies führt, zusammen mit der Schrägstellung von P und O zur optischen Achse dazu, dass das geschriebene Hologramm eine elliptische Form besaß. Dies ist in Figur 2 dargestellt.

Da es sich bei O um einen diffusen Reflektor handelte, war das Hologramm durch Beleuchten mit einer Punktlichtquelle (z. B. Taschenlampe oder LED-Leuchte) leicht zu rekonstruieren. In diesem Fall enthält die Photopolymer-Formulierung einen aktiven Photoinitiator. Dieser Befund ist der Tabelle 2 mit (J) gekennzeichnet.

Nach dem Schreiben des Hologramms wurde das Photopolymer unter UV-Strahlung geblichen. Dazu wurden die Proben aus einer lichtecht verpackten Aluminiumtüte mit der Glasseite nach oben auf das Förderband einer UV-Anlage gelegt und mit einer Bandgeschwindigkeit von 2.5 m/min zweimal unter einer Lampe des Typs Fusion UV 558434 KR 85 mit einer nominalen Leistungsdichte von 80 $W/cm^2$ mit einer Energiedichte auf dem Photopolymer von ~2 $J/cm^2$ belichtet.

## Prüfung der Eignung als Photoinitiatorsvstem gemäß dem erfindungsgemäßen Verfahren

Beispiel 1

Es wird angenommen, dass für eine gegebene Aufgabe die Photopolymerformulierung mit einem HeNe Laser mit einer Wellenlänge von 630 nm belichtet werden soll. Es wird nun mit Hilfe des erfindungsgemäßen Verfahrens die Eignung des Zweikomponenten-Initiatorsystems New Methylene blue (NMB F7)/CGI909 für die beschriebene Anwendung überprüft.

Der Reaktionsmechanismus des New Methylene Blue (NMB/F7)/CGI909 Zweikomponenten-Initiatorsystems besteht aus den folgenden Schritten:

1. Lichtabsorption des Farbstoffs. Dabei Übergang vom elektronischen Grundzustand ($S_0$) in den ersten angeregten Singulett-Zustand ($S_1$).
2. Intersystem Crossing in einen Triplett Zustand des Farbstoffs und schnelle Relaxation zum niedrigsten Triplett Zustand ($T_1$).
3. Reaktion des Farbstoff-Moleküls im $T_1$-Zustand mit CGI909 unter Bildung eines neutralen NMB-Radikals $NMB_{rad}$, eines Borans $BAr_3$ sowie eines Hexylradikals $Hex_{rad}$ entsprechend der folgenden Reaktionsgleichung:

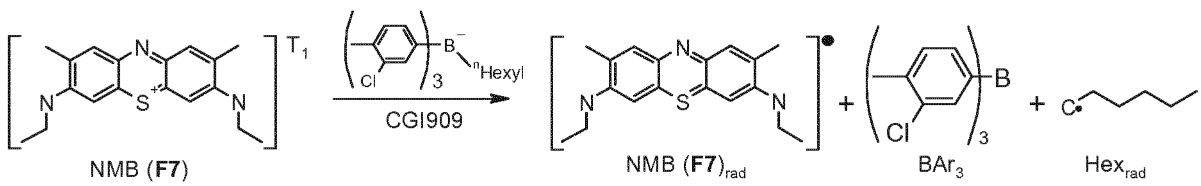


NMB (**F7**) NMB (**F7**)$_{rad}$ $BAr_3$ $Hex_{rad}$

Die dreidimensionalen Strukturen von New Methylene Blue und CGI909 sowie der Folgeprodukte von CGI909 wurden einer Konformerenanalyse mit Hilfe des *conformers* Modul aus dem Programmpaket Materials Studio der Firma Accelrys unterzogen. Das Ergebnis dieser Analyse war, dass im vorliegenden Fall keine Betrachtung von mehr als jeweils dem stablisten Konformer erforderlich ist, da die Moleküle, mit Ausnahme des Hexylradikals, sehr rigide sind.

Als Ausgangpunkt für die quantenchemischen Rechnungen wurde daher jeweils das, nach der im Rahmen der Konformerenanalyse vorgenommenen Kraftfeldrechnung, stabliste Konformer verwendet.

Die auf diese Weise gefundenen jeweils stablisten Konformere wurden dann entsprechend dem Schritt d) des erfindungsgemäßen Verfahrens im elektronischen Grundzustand quantenchemisch geometrieoptimiert. Zu diesem Zweck wurde das Programmpaket TURBOMOLE Version 6.1 verwendet. Die Rechnungen erfolgten auf dichtefunktionaltheoretischem Niveau, wobei das Dichtefunktional BP86 und der Basissatz TZVP zum Einsatz kamen. Weiterhin wurde das *COnductor like Screnning MOdel* (COSMO) Kontinuumsolventmodell verwendet. Die so erhaltenen Molekülgeometrien wurden dann Einzelpunktrechnungen auf BH-LYP/TZVP+COSMO Niveau unterzogen. Das Ergebnis dieser Rechnung, also absolute elektronische Energien auf DFT(BH-LYP/TZVP+COSMO//BP86/TZVP+COSMO) Niveau (siehe

Tabelle 1), wird, wie weiter unten beschrieben, zur Berechnung der Reaktionsenergien der Initiationsreaktion verwendet.

Tabelle 1: Absolute elektronische Grundzustandsenergien der an der NMB(F7)/CGI909 Initiationsreaktion beteiligten Verbindungen

| Verbindung | E(BH-LYP/TZVP+COSMO//BP86/TZVP+COSMO)/Hartree |
|---|---|
| CGI909 | -2452.841810 |
| $NMB(F7)_{rad}$ | -1261.354348 |
| $BPh_3$ | -2216.337044 |
| $Hex_{rad}$ | -236.3315548 |

Im nächsten Schritt (Schritt e)) erfolgte die Berechnung der elektronischen Anregungsenergie von New Methylene blue mit Hilfe der zeitabhängigen Dichtefunktionaltheorie und unter Verwendung des BH-LYP Hybridfunktionals und des TZVP Basissatzes sowie des COSMO Modells. Die Anregungsenergie ergab sich zu 2.53 eV und die Oszillatorenstärke (gemäß der Längenform des Dipoloperators) zu 1.08 Einheiten. Die Anregungsenergie wird um ihren, empirisch an einem großen Benchmarksatz an Farbstoffen ermittelten, systematischen Fehler von 0.56 eV auf 1.97 eV korrigiert, was einer Wellenlänge von 623 nm entspricht.

Als letzte verbliebende zu berechnende Energie wird die absolute Energie des ersten Triplett-Zustands von Neumethylenblau berechnet. Dies erfolgte ebenfalls auf dem DFT(BH-LYP/TZVP+COSMO// BP86/TZVP+COSMO) Niveau und ergab eine Energie von $E(^3NMB)$ = -1261.164863 Hartree.

Das Intersystem Crossing des Farbstoffs vom $S_0$ in den $T_1$-Zustand ist ausnahmslos negativ. Damit ist für das Zweikomponenten-Initiatorsystem aus dem oben beschriebenen Reaktionsmechanismus nur die Reaktion von Neumethylenblau im Triplett Zustand mit CGI909 zu den entsprechenden Fragmenten für die thermodynamische Relevanz entscheidend. Diese ergibt sich aus den berechneten absoluten Energien wie folgt:

$$\begin{aligned} E_{Coin} &= E(NMB_{rad}) + E(Hex_{rad}) + E(BAr_3) - E(^3NMB) - E(CGI909) \\ &= (-1261.354348 - 236.3315548 - 2216.337044 + 1261.164863 + 2452.841810)\ \text{Hartree} \\ &= -0.016274\ \text{Hartree} \\ &\approx \mathbf{-43\ kJ/mol}. \end{aligned}$$

Da die in diesem Beispiel einzige relevante Reaktionsenergie $E_{Coin}$ negativ ist, die berechnete Absorptionsenergie mit 630 nm perfekt mit dem Zielwert von 630 nm übereinstimmt und die Anregung mit einer Oszillatorenstärke von 1.08 Einheiten über dem Schwellenwert von 0.2 liegt, ist das Zweikomponenten-Initiatorsystem NMB/CGI909 im Sinne des erfindungsgemäßen Verfahrens als geeignet einzustufen.

Das Initiatorsystem erwies sich in Übereinstimmung mit der Vorhersage als holographisch aktiv und auch die berechnete Absorptionswellenlänge von 630 nm stimmt gut mit dem experimentellen Wert von 623 nm überein.

Analog zu Beispiel Medium 1 wurden Medien mit den jeweiligen in der Tabelle 2 angegebenen Farbstoffen hergestellt und geprüft, die erhaltenen Ergebnisse des erfindungsgemäßen Verfahrens sind ebenfalls Tabelle 2 zu entnehmen.

Tabelle 2: Theoretische und experimentelle Absorptionsenergien, theoretische Absorptionsstärken ($f^l$) sowie Coinitiationsenergien (siehe Text) und experimentelle holographische Aktivität von Farbstoffen in Zweikomponenten-Initiatorsystemen mit CGI909.

| Farbstoff-beispiel | Name | Absorption/eV | | Holograpisch Aktiv (J/N) | E(CGI)/ (kJ/mol) | Struktur |
|---|---|---|---|---|---|---|
| | | Theorie ($E/f^l$) | Exp. | | | |
| F2 | Ketocoumarin | 2.64 $f^l$ = 1.90 | 2.70 | J | -67 | 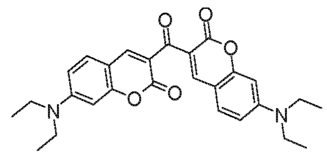 |
| F3 | Carbolfuchsin | 2.41 $f^l$ = 0.79 | 2.24 | J | -67 | 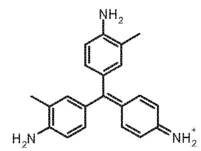 |
| F4 | Rhodamin 6G | 2.36 $f^l$ = 1.17 | 2.37 | J | -66 | 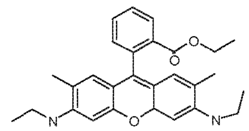 |
| F5 | | 2.22 $f^l$ = 0.48 | 2.17 | J | -60 | 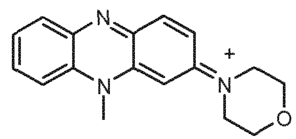 |
| F6 | | 2.10 $f^l$ = 1.05 | 2.13 | J | -59 | 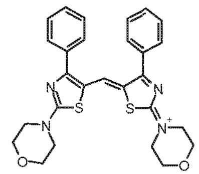 |
| F7 | New Methylen Blue | 1.97 $f^l$ = 1.08 | 1.99 | J | -43 | 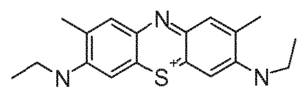 |

(fortgesetzt)

| Farbstoff-beispiel | Name | Absorption/eV | | Holograpisch Aktiv (J/N) | E(CGI)/ (kJ/mol) | Struktur |
|---|---|---|---|---|---|---|
| | | Theorie (E/$f^l$) | Exp. | | | |
| F8 | Kristallviolett | 2.26 $f^l$ = 0.98 | 2.10 | J | -42 | 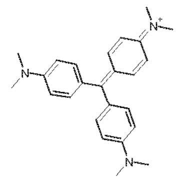 |
| F9 | | 2.64 f = 1.00 | 2.64 | J | -41 | 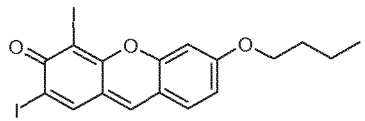 |
| F10 | Safranin O | 2.37 $f^l$ = 0.97 | 2.32 | J | -40 | 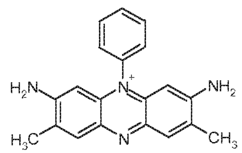 |
| F11 | | 2.10 $f^l$ = 1.22 | 2.23 | J | -40 | 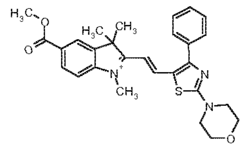 |
| F12 | Brilliant Green | 2.08 $f^l$ = 1.20 | 1.98 | J | -38 | 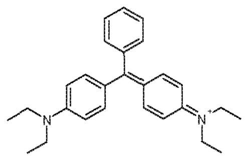 |
| F13 | C. I. Basic Red 14 | 2.34 $f^l$ = 1.64 | 2.37 | J | -36 | 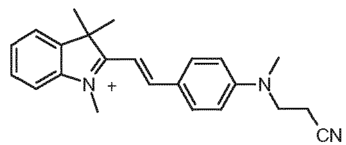 |

(fortgesetzt)

| Farbstoff-beispiel | Name | Absorption/eV | | Holograpisch Aktiv (J/N) | E(CGI)/ (kJ/mol) | Struktur |
|---|---|---|---|---|---|---|
| | | Theorie (E/$f^l$) | Exp. | | | |
| F14 | C. I. Basic Orange 21 | 2.49 $f^l$ = 1.13 | 2.53 | J | -36 | 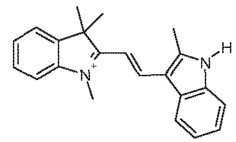 |
| F15 | | 2.26 $f^l$ = 1.89 | 2.24 | J | -34 | 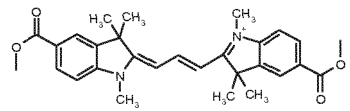 |
| F16 | C. I. Basic Blue 3 | 1.94 $f^l$ = 1.30 | 1.89 | J | -33 | 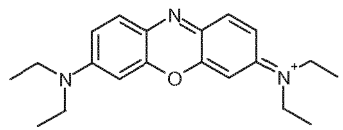 |
| F17 | Ethylviolett | 2.24 $f^l$ = 1.02 | 2.08 | J | -30 | 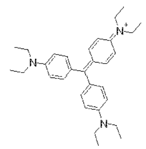 |
| F18 | | 2.62 $f^l$ = 1.33 | 2.52 | J | -26 | 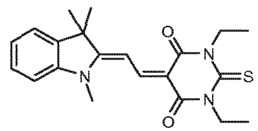 |
| F19 | EAB | 3.29 $f^l$ = 1.14 | 3.53 | J | -23 | 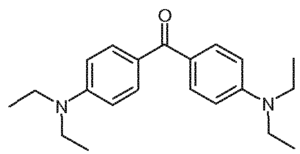 |

(fortgesetzt)

| Farbstoff-beispiel | Name | Absorption/eV | | Holograpisch Aktiv (J/N) | E(CGI)/ (kJ/mol) | Struktur |
|---|---|---|---|---|---|---|
| | | Theorie (E/$f^l$) | Exp. | | | |
| F20 | C. I. Basic Violet 16 | 2.28 $f^l$ = 1.69 | 2.19 | J | -18 | 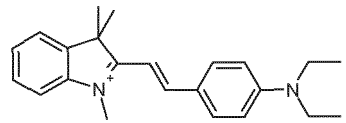 |
| F21 | | 2.65 $f^l$ = 1.46 | 2.58 | J | -14 | 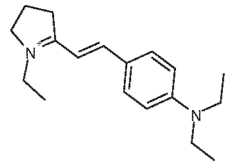 |
| F22 | | 2.39 $f^l$ = 1.62 | 2.52 | N | 3 | 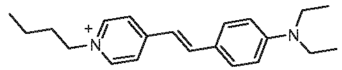 |
| F23 | C. I. Basic Yellow 28 | 2.57 $f^l$ = 1.10 | 2.76 | N | 5 | 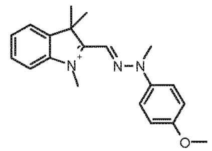 |
| F24 | | 2.47 $f^l$ = 1.56 | 2.51 | N | 7 | 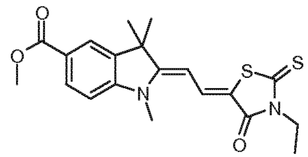 |
| F25 | Pinacyanol | 2.05 $f^l$ = 2.03 | 2.05 | N | 7 | 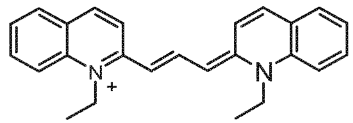 |

(fortgesetzt)

| Farbstoff-beispiel | Name | Absorption/eV | | Holograpisch Aktiv (J/N) | E(CGI)/ (kJ/mol) | Struktur |
|---|---|---|---|---|---|---|
| | | Theorie (E/f[l]) | Exp. | | | |
| F26 | | 2.62 f[l] = 1.52 | 2.64 | N | 21 | 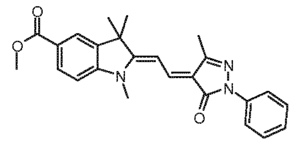 |
| F29 | | 2.04 f[l] = 2.05 | 1.95 | N | 1 | 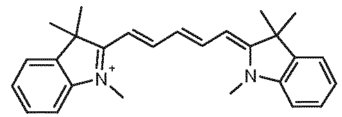 |
| F30 | | 2.00 f[l] = 2.34 | 1.91 | J | -17 | 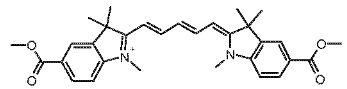 |

Farbstoffbeispiel F5 wurde analog zu EP 0 671 393 hergestellt.

Farbstoffbeispiel F6 wurde gemäß Ronald Flaig, Dissertation 1996 hergestellt.

Farbstoffbeispiel 9 ist aus der WO 9514689 bekannt und im Jahr 2012 bei Spectra Group Limited, Inc, 27800 Lemoyne Road Suite J, Millbury, OH 43447, USA erhältlich.

Farbstoffbeispiel F11 wurde analog zu US 3 573 289 hergestellt.

Farbstoffbeispiel F15 wurde analog zu EP 58 863 hergestellt.

Farbstoffbeispiele F18, F24 und F26 wurden analog zu Synthesis, 1999, 2103 hergestellt.

Farbstoffbeispiel F21 wurde analog zu DE 883 025 hergestellt.

Farbstoffbeispiel F22 wurde gemäß J. Chem. Soc. 1938, 1454 hergestellt.

Farbstoffbeispiel F29 wurde gemäß Proceedings of the Imperial Academy (Tokyo), 1932, vol. 8, p. 421 (Chem. Zentralbl., 1934, vol. 105, # II p. 2227) hergestellt.

Farbstoffbeispiel F30 wurde analog Beispiel 29 hergestellt.

<u>Beispiel 2</u>

Es wird angenommen, dass für eine gegebene Aufgabe eine Photopolymerformulierung mit Laserlicht der Wellenlänge von 530 nm belichtet werden soll. Es wird nun mit Hilfe des erfindungsgemäßen Verfahrens die Eignung des Dreikomponenten Initiatorsystems Farbstoff F1/Irgacure® 250/EDB für die beschriebene Anwendung überprüft.

Der Reaktionsmechanismus des Farbstoffs F1/Irgacure® 250/EDB Dreikomponenten-Initiatorsystems besteht aus den folgenden Schritten:

1. Lichtabsorption des Farbstoffs. Dabei Übergang vom elektronischen Grundzustand ($S_0$) in den ersten angeregten Singulett-Zustand ($S_1$).
2. Intersystem Crossing in einen Triplett Zustand des Farbstoffs und schnelle Relaxation zum niedrigsten Triplett Zustand ($T_1$).
3. Reaktion des Farbstoff-Moleküls im $T_1$-Zustand mit Irgacure® 250. Dabei Bildung von Methyl 1-Iodo-4-isobutyl-benzol IPh$^i$Bu und einem Toluyl-Radikal.
4. Reaktion des Toluyl-Radikals $Tol_{rad}$ mit Ethyl-4-dimethylaminobenzoat (EDB) zu Toluol und dem entsprechenden EDB-Radikal $EDB_{rad}$.

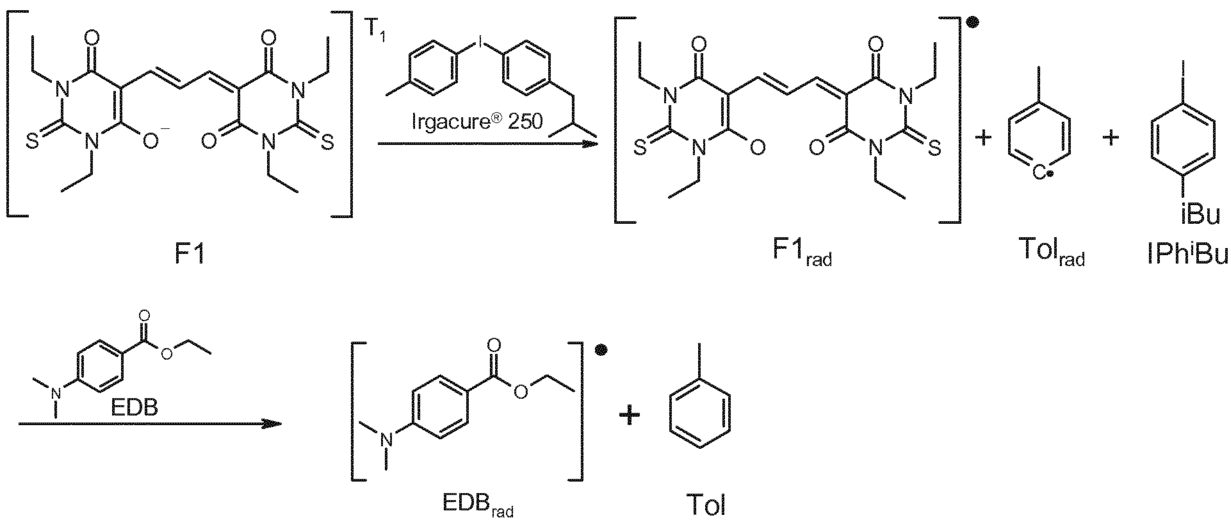


**Reaktionsmechanismus des Farbstoffs F1/Irgacure® 250/EDB Dreikomponenten-Initiatorsystems**

Die dreidimensionalen Strukturen von Farbstoff F1, Irgacure® 250, EDB, sowie der Zersetzungsprodukte von Irgacure® 250 und dem durch H-Abstraktion entstehenden aus EDB abgeleiteten Radikal wurden einer Konformerenanalyse mit Hilfe des *conformers* Modul aus dem Programmpaket Materials Studio der Firma Accelrys unterzogen. Das Ergebnis dieser Analyse war, dass im vorliegenden Fall keine Betrachtung von mehr als jeweils dem stablisten Konformer erforderlich ist. Als Ausgangpunkt für die quantenchemischen Rechnungen wurde daher jeweils das, nach der im Rahmen der Konformerenanalyse vorgenommenen Kraftfeldrechnung, stabilste Konformer verwendet.

Die auf diese Weise gefundenen jeweils stabilsten Konformere wurden dann entsprechend dem Schritt d) des erfindungsgemäßen Verfahrens im elektronischen Grundzustand quantenchemisch geometrieoptimiert. Zu diesem

Zweck wurde das Programmpaket TURBOMOLE Version 6.1 verwendet. Die Rechnungen erfolgten auf dichtefunktionaltheoretischem Niveau, wobei das Dichtefunktional BP86 und der Basissatz TZVP zum Einsatz kamen. Weiterhin wurde das *COnductor like Screnning MOdel* (COSMO) Kontinuumsolventmodell verwendet. Die so erhaltenen Molekülgeometrien wurden dann Einzelpunktrechnungen auf BH-LYP/TZVP+COSMO Niveau unterzogen. Das Ergebnis dieser Rechnung, also absolute elektronische Energien auf DFT(BH-LYP/TZVP+COSMO//BP86/TZVP+COSMO) Niveau (siehe Tabelle 3), wird, wie weiter unten beschrieben, zur Berechnung der Reaktionsenergien der Initiationsreaktion verwendet.

Tabelle 3: Absolute elektronische Grundzustandsenergien der an der Farbstoff F1/Irgacure® 250/EDB Initiationsreaktion beteiligten Verbindungen

| Verbindung | E(BH-LYP/TZVP+COSMO//BP86/TZVP+COSMO)/Hartree |
|---|---|
| Irgacure® 250 | -670.781969 |
| Farbstoff $F1_{rad}$ | -2053.882516 |
| $Tol_{rad}$ | -270.795918 |
| $IPh^iBu$ | -400.140492 |
| EDB | -633.254506 |
| $EDB_{rad}$ | -632.603044 |
| Tol | -271.481294 |

Im nächsten Schritt (Schritt e)) erfolgte die Berechnung der elektronischen Anregungsenergie von F1 mit Hilfe der zeitabhängigen Dichtefunktionaltheorie und unter Verwendung des BH-LYP Hybridfunktionals und des TZVP Basissatzes sowie des COSMO Modells. Die Anregungsenergie ergab sich zu 2.98 eV und die Oszillatorenstärke (gemäß der Längenform des Dipoloperators) zu 1.63 Einheiten. Die Anregungsenergie wird um ihren, empirisch an einem großen Benchmarksatz an Farbstoffen ermittelten, systematischen Fehler von 0.56 eV auf 2.42 eV korrigiert, was einer Wellenlänge von 512 nm entspricht.

Als letzte verbliebende zu berechnende Energie wird die absolute Energie des ersten Triplett-Zustands von F1 berechnet. Dies erfolgt ebenfalls auf dem DFT(BH-LYP/TZVP+COSMO// BP86/TZVP+COSMO) Niveau und ergab eine Energie von $E(^3F1)$ = -2054.000848 Hartree.

Das Intersystem Crossing des Farbstoffs vom $S_0$ in den $T_1$-Zustand ist ausnahmslos negativ. Damit ist für das Dreikomponenten-Initiatorsystem gemäß dem oben beschriebenen Reaktionsmechanismus nur die Reaktion von Farbstoff F1 mit Irgacure® 250 ($E_{Coin,1}$), sowie die anschließende Reaktion des aus der Irgacure® 250 Zersetzung entstehenden Toluyl-Radikal mit EDB ($E_{Coin,2}$) relevant. Diese ergeben sich aus den berechneten absoluten Energien wie folgt:

$$\begin{aligned} E_{Coin,1} &= E(F1_{rad}) + E(Tol_{rad}) + E(IPh^iBu) - E(^3F1) - E(\text{Irgacure® } 250) \\ &= (-2053.882516 - 270.795918 - 400.140492 + 2054.000848 + 670.781969)\ \text{Hartree} \\ &= -0.036109\ \text{Hartree} \\ &\approx \mathbf{-95\ kJ/mol} \end{aligned}$$

$$\begin{aligned} E_{Coin,2} &= E(EDB_{rad}) + E(Tol) - E(Tol_{rad}) - E(EDB) \\ &= (-632.603044 - 271.481294 + 270.795918 + 633.254506)\ \text{Hartree} \\ &= -0.033913\ \text{Hartree} \\ &\approx \mathbf{-89\ kJ/mol} \end{aligned}$$

Da beide Reaktionsenergien negativ sind, die berechnete Absorptionsenergie mit 512 nm im Toleranzinterval von ±50 nm um den Zielwert von 530 nm liegt und die Anregung mit einer Oszillatorenstärke von 1.63 Einheiten über dem Schwellenwert von 0.2 liegt, ist das Dreikomponenten-Initiatorsystem Farbstoff F1/Irgacure® 250 /EDB entsprechend dem Schritt h) des erfindungsgemäßen Verfahrens als geeignet einzustufen.

Das Photoinitiatorsystem erwies sich in Übereinstimmung mit der Vorhersage als holographisch aktiv und auch die berechnete Absorptionswellenlänge von 512 nm stimmt gut mit dem experimentellen Wert von 531 nm überein.

Weitere auf analoge Weise berechnete Beispiele sind Tabelle 4 zu entnehmen. Dabei wurde $E_{Coin,2}$ nicht mit angegeben, da dieses unabhängig vom Farbstoff ist und daher für alle Dreikomponenten-Photoinitiatorsysteme dem Beispiel 2 entspricht. Analog zu Beispiel Medium 2 wurden Medien mit den jeweiligen in der Tabelle 4 angegebenen Farbstoffen hergestellt und geprüft, die erhaltenen Ergebnisse des erfindungsgemäßen Verfahrens sind ebenfalls Tabelle 2 zu entnehmen.

Tabelle 4: Theoretische und experimentelle Absorptionsenergien, sowie Coinitiationsenergien (siehe Text) und experimentelle holographische Aktivität von Farbstoffen in Dreikomponenten-Initiatorsystemen mit Irgacure® 250 und EDB.

| Farbstoff-beispiel | Name | Absorption/eV | | Holographisch Aktiv (J/N) | $E_{Coin,1}$/ (kJ/mol) | Struktur |
|---|---|---|---|---|---|---|
| | | Theorie | Exp. | | | |
| F1 | | 2.42 $f^{l}$ = 1.63 | 2.33 | J | -95 | 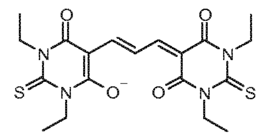 |
| F2 | Ketocoumarin | 2.64 $f^{l}$ = 1.90 | 2.70 | J | -131 | 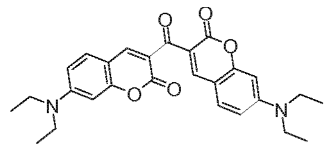 |
| F3 | Carbolfuchsin | 2.41 $f^{l}$ = 0.79 | 2.24 | N | 0 | 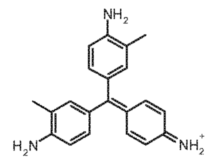 |
| F9 | | 2.64 $f^{l}$ = 1.00 | 2.64 | J | -31 | 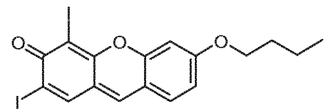 |
| F10 | Safranin O | 2.37 $f^{l}$ = 0.97 | 2.32 | J | -33 | 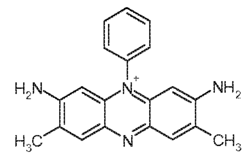 |
| F18 | | 2.62 $f^{l}$ = 1.33 | 2.52 | J | -84 | 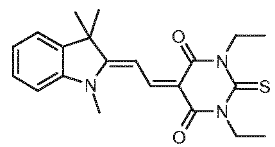 |

(fortgesetzt)

| Farbstoff-beispiel | Name | Absorption/eV | | Holographisch Aktiv (J/N) | $E_{Coin,1}$/ (kJ/mol) | Struktur |
|---|---|---|---|---|---|---|
| | | Theorie | Exp. | | | |
| F19 | EAB | 3.29 $f^{l}$ = 1.14 | 3.53 | J | -187 | 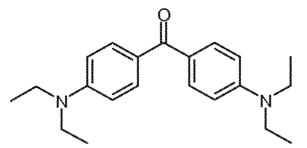 |
| F27 | | 2.33 $f^{l}$ = 1.80 | 2.22 | J | -99 | 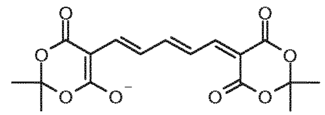 |
| F28 | | 2.11 $f^{l+}$ = 0.63 | 1.95 | N | 182 | 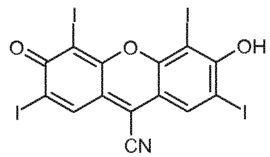 |

Farbstoffbeispiel 27 wurde analog zu EP 1 253 148 hergestellt.

Farbstoffbeispiel 28 ist aus der WO 9514689 bekannt und im Jahr 2012 bei Spectra Group Limited, Inc, 27800 Lemoyne Road Suite J, Millbury, OH 43447, USA erhältlich.

Güte der Vorhersage der Anregungsenergien die betrachteten Beispiele:

Für die oben ausführlich beschriebenen Beispiele 1 und 2 sind die Fehler bei der Vorhersage von $\lambda_{max}$ mit 0.02 eV (Beispiel 1) und 0.09 eV (Beispiel 2) klein. Wie Figur 3 zu entnehmen ist, gilt dies auch für die übrigen Beispielfarbstoffe.

Die Anregungsenergie von EAB wird mit 0.24 eV unterschätzt, was den Maximalfehler innerhalb des hier untersuchten Beispielsatzes darstellt, und die mittlere quadratische Abweichung beträgt 0.1 eV. Diese sehr kleinen Werte zeigen, dass die Genauigkeit der Methode ausreichend hoch ist, um sie im Rahmen des erfindungsgemäßen Verfahrens einzusetzen.

## Patentansprüche

1. Computer-implementiertes Verfahren zur Auswahl von Photoinitiatorsystemen für Photopolymerformulierungen, bei dem

   a) ein Photoinitiatorsystem, umfassend mindestens einen Sensibilisator und mindestens einen Coinitiator, ausgewählt wird,
   b) der Reaktionsmechanismus des Photoinitiatorsystems durch Aufführung der konkreten Reaktionssequenz unter Berücksichtigung der Redox-Eigenschaften der beteiligten Verbindungen aufgestellt wird, welcher den Übergang des Sensibilisators oder der Sensibilisatoren in einen elektronisch angeregten Zustand beziehungsweise elektronisch angeregte Zustände durch Absorption elektromagnetischer Strahlung und in Folgeschritten die als Initiationsreaktion bezeichnete Reaktion des Sensibilisators im elektronisch angeregten Zustand oder der Sensibilisatoren in elektronisch angeregten Zuständen mit dem Coinitiator oder den Coinitiatoren zu mindestens einem Radikal und weiteren, vom jeweiligen Photoinitiatorsystem abhängigen, Produkten, beinhaltet,
   c) die dreidimensionale Molekülgeometrien des Sensibilisators oder der Sensibilisatoren, des Coinitiators oder der Coinitiatoren, sowie aller durch den Reaktionsmechanismus definierten Zwischenstufen und Endprodukte der Initiationsreaktion generiert werden und diese dann einer Konformerenanalayse auf Basis eines Kraftfeldverfahrens unterzogen werden,
   d) die Molekülgeometrien der Strukturen aus Schritt c) mit der jeweils niedrigsten relativen Kraftfeldenergie quantenchemisch im elektronischen Grundzustand optimiert werden und die absoluten elektronischen Energien der optimierten Strukturen bestimmt werden,
   e) die Anregungsenergien und Oszillatorenstärken des elektronischen Absorptionsspektrums des Sensibilisators oder der Sensibilisatoren mit Hilfe des quantenchemischen zeitabhängigen Dichtefunktionaltheorieverfahrens berechnet werden und die Anregungsenergien um ihren systematischen Fehler korrigiert werden,
   f) die Molekülgeometrien aller Sensibilisatoren in den bezüglich der Coinitiationsreaktion relevanten, angeregten elektronischen Zuständen auf dichtefunktional-theoretischem Niveau optimiert werden und die absoluten elektronischen Energien bestimmt werden,
   g) die Reaktionsenergien alle Teilreaktionen des im Schritt b) aufgestellten Mechanismus berechnet werden, und
   h) das Photoinitiatorsystem als geeignet eingestuft wird, wenn die im Schritt e) bestimmten Anregungsfrequenzen in einem $\pm 50$ nm Intervall um die für die Belichtung vorgesehene Lichtwellenlänge liegt und eine Oszillatorenstärke größer als 0.2 aufweist und wenn gleichzeitig alle unter g) berechneten Reaktionsenergien negativ sind.

2. Verfahren nach Anspruch 1 **dadurch gekennzeichnet, dass** in den Schritten d) und f) die quantenchemischen Geometrieoptimierungen mit Hilfe des DFT, BP86/TZVP Verfahrens vorgenommen und anschließend DFT BH-LYP/TZVP Einzelpunktrechnung durchgeführt werden,

3. Verfahren nach einem der Ansprüche 1 oder 2 **dadurch gekennzeichnet, dass** die quantenchemischen Rechnungen unter Verwendung des *COnductor like Screening MOdels* (COSMO) durchgeführt werden.

4. Verfahren nach einem der Ansprüche 1 bis 3 **dadurch gekennzeichnet, dass** im Schritt e) die zeitabhängige DFT BH-LYP/TZVP Methode zur Berechnung der Absorptionsspektren verwendet wird.

**5.** Verfahren nach Anspruch 4 **dadurch gekennzeichnet, dass** die zeitabhängige DFT Rechnung unter Verwendung des *COnductor like Screening MOdels* (COSMO) durchgeführt werden.

**6.** Verfahren nach einem der Ansprüche 1 bis 5 **dadurch gekennzeichnet, dass** in den Schritten d) und f) an Stelle nur der Molekülgeometrie mit der niedrigsten Kraftfeldenergie alle Molekülgeometrien in einem Energiefenster von 0-8 kJ/mol berücksichtigt werden und sowohl für die Berechnung des Absorptionsspektrums des Sensibilisators im Schritt e) als auch für die Bestimmung der Reaktionsenergien im Schritt g) Boltzmann gewichtete Mittelwerte der Anregungsenergien, Absorptionsstärken, sowie Gesamtenergien verwendet werden, wobei die Boltzmann-Gewichte auf dichtefunktionaltheoretischem Niveau, besonders bevorzugt auf Basis von DFT BP86/TZVP Geometrieoptimierungen mit anschließenden DFT BH-LYP/TZVP Einzelpunktrechnungen jeweils unter Verwendung von COSMO, berechnet werden.

**7.** Verfahren nach einem der Ansprüche 1 bis 6 **dadurch gekennzeichnet, dass** im Schritt e) der systematische Fehler mit +0.7 Elektronenvolt angenommen wird.

**8.** Verfahren nach einem der Ansprüche 1 bis 6 **dadurch gekennzeichnet, dass** im Schritt e) der systematische Fehler mit +0.56 Elektronenvolt angenommen wird.

## Claims

**1.** Computer-implemented method for selecting photoinitiator systems for photopolymer formulations, wherein

a) a photoinitiator system comprising at least one sensitizer and at least one coinitiator (3) is selected,
b) the reaction mechanism of the photoinitiator system is established by specifying the concrete reaction sequence taking account of the redox properties of the compounds involved, said reaction mechanism including the transition of the sensitizer or sensitizers into an electronically excited state or, respectively, electronically excited states by absorption of electromagnetic radiation and in subsequent steps the reaction - referred to as initiation reaction - of the sensitizer in the electronically excited state or the sensitizers in electronically excited states with the coinitiator or coinitiators to form at least one radical and further products, which are dependent on the respective photoinitiator system,
c) the three-dimensional molecular geometries of the sensitizer or sensitizers, of the coinitiator or coinitiators and also of all intermediates and end products of the initiation reaction that are defined by the reaction mechanism are generated and then subjected to a conformer analysis on the basis of a force field method,
d) the molecular geometries of the structures from step c) having the lowest relative force field energy in each case are optimized quantum-chemically in the electronic ground state and the absolute electronic energies of the optimized structures are determined,
e) the excitation energies and oscillator strengths of the electronic absorption spectrum of the sensitizer or sensitizers are calculated with the aid of the quantum-chemical time-dependent density functional theory method and the excitation energies are corrected for their systematic error,
f) the molecular geometries of all sensitizers in the excited electronic states relevant with regard to the coinitiation reaction are optimized on a density functional theoretical level and the absolute electronic energies are determined,
g) the reaction energies of all partial reactions of the mechanism established in step b) are calculated, and
h) the photoinitiator system is classified as suitable if the excitation frequencies determined in step e) are in a +/- 50 mm interval around the lightwave length provided for the exposure and have an oscillator strength greater than 0.2 and if at the same time all reaction energies calculated under g) are negative.

**2.** Method according to Claim 1, **characterized in that** in steps d) and f) the quantum-chemical geometry optimizations are performed with the aid of the DFT, BP86/TZVP method and then DFT BH-LYP/TZVP single-point calculations are carried out.

**3.** Method according to either of Claims 1 or 2, **characterized in that** the quantum-chemical calculations are carried out using the *COnductor like Screening MOdel* (COSMO).

**4.** Method according to any of Claims 1 to 3, **characterized in that** in step e) the time-dependent DFT BH-LYP/TZVP method is used for calculating the absorption spectra.

5. Method according to Claim 4, **characterized in that** the time-dependent DFT BH-LYP/TZVP calculation is carried out using the *COnductor like Screening MOdel* (COSMO).

6. Method according to any of Claims 1 to 5, **characterized in that** in steps d) and f), instead of only the molecular geometry having the lowest force field energy, all molecular geometries in an energy window of 0-8 kJ/mol are taken into account and Boltzmann-weighted mean values of the excitation energies, absorption strengths, and total energies are used both for the calculation of the absorption spectrum of the sensitizer in step e) and for the determination of the reaction energies in step g), wherein the Boltzmann weights are calculated on a density functional theoretical level, particularly preferably on the basis of DFT BP86/TZVP geometry optimizations with subsequent DFT BH-LYP/TZVP single-point calculations using COSMO in each case.

7. Method according to any of Claims 1 to 6, **characterized in that** in step e) the systematic error is assumed to be +0.7 electronvolt.

8. Method according to any of Claims 1 to 6, **characterized in that** in step e) the systematic error is assumed to be +0.56 electronvolt.

## Revendications

1. Procédé mis en oeuvre par ordinateur de sélection de systèmes photoinitiateurs pour des formulations de photopolymères, selon lequel

    a) un système photoinitiateur, comprenant au moins un sensibilisateur et au moins un co-initiateur, est sélectionné,
    b) le mécanisme de réaction du système photoinitiateur est établi en exécutant la séquence de réaction concrète en tenant compte des propriétés redox des composés participants, laquelle comprend la transition du sensibilisateur ou des sensibilisateurs dans un état excité électroniquement ou dans des états excités électroniquement par absorption de rayonnement électromagnétique et, dans les étapes suivantes, la réaction désignée comme réaction d'initiation du sensibilisateur dans l'état excité électroniquement ou des sensibilisateurs dans les états excités électroniquement avec le co-initiateur ou les co-initiateurs pour obtenir au moins un radical et des produits supplémentaires dépendants du système photoinitiateur respectif,
    c) les géométries moléculaires tridimensionnelles du sensibilisateur ou des sensibilisateurs du co-initiateur ou des co-initiateurs, ainsi que tous les étages intermédiaires définis par le mécanisme de réaction et les produits finaux de la réaction d'initiation sont générés et ceux-ci sont ensuite soumis à une analyse de conformité sur la base d'un procédé de champ de force,
    d) les géométries moléculaires des structures obtenues à l'étape c) ayant l'énergie de champ de force respectivement la plus basse sont optimisées par chimie quantique à l'état électronique fondamental et les énergies électroniques absolues des structures optimisées sont déterminées,
    e) les énergies d'excitation et les intensités d'oscillateur du spectre d'absorption électronique du sensibilisateur ou des sensibilisateurs sont calculées à l'aide du procédé en chimique quantique de la théorie de la fonctionnelle de la densité en fonction du temps et les énergies d'excitation sont corrigées de leurs erreurs systématiques,
    f) les géométries moléculaires de tous les sensibilisateurs sont optimisées au niveau théorique de la fonctionnelle de la densité dans les états excités électroniquement pertinents pour ce qui concerne la réaction de co-initiation et les énergies électroniques absolues sont déterminées,
    g) les énergies de réaction de toutes les réactions partielles du mécanisme établi à l'étape b) sont calculées, et
    h) le système photoinitiateur est classifié comme étant adapté lorsque les fréquences d'excitation déterminées à l'étape e) se trouvent dans un intervalle de $\pm$50 nm autour de la longueur d'onde lumineuse prévue pour l'illumination et possèdent une intensité d'oscillateur supérieure à 0,2 et lorsque simultanément, toutes les énergies de réaction calculées à l'étape g) sont négatives.

2. Procédé selon la revendication 1, **caractérisé en ce que** dans les étapes d) et f), les optimisations géométriques par chimie quantique sont réalisées à l'aide du procédé DFT, BP86/TZVP et un calcul de point individuel DFT BH-LYP/TZVP est ensuite effectué.

3. Procédé selon l'une des revendications 1 ou 2, **caractérisé en ce que** les calculs chimiques quantiques sont réalisés en utilisant les modèles COSMO (COnductor like Screening MOdels).

4. Procédé selon l'une des revendications 1 à 3, **caractérisé en ce que** dans l'étape e), la méthode DFT BH-LYP/TZVP dépendante du temps est utilisée pour calculer les spectres d'absorption.

5. Procédé selon la revendication 4, **caractérisé en ce que** le calcul de DFT en fonction du temps est réalisé en utilisant les modèles COSMO (COnductor like Screening MOdels).

6. Procédé selon l'une des revendications 1 à 5, **caractérisé en ce que** dans les étapes d) et f), au lieu de seule la géométrie moléculaire ayant l'énergie de champ de force la plus basse, toutes les géométries moléculaires dans une fenêtre d'énergie de 0 à 8 kJ/mol sont prises en compte et les valeurs moyennes pondérées par la méthode Boltzmann des énergies d'excitation, des intensités d'absorption ainsi que des énergies totales sont utilisées à la fois pour le calcul du spectre d'absorption du sensibilisateur à l'étape e) et pour la détermination des énergies de réaction à l'étape g), les poids Boltzmann étant calculés au niveau théorique de la fonctionnelle de la densité, notamment de préférence sur la base des optimisations géométriques DFT BP86/TZVP suivies des calculs de points individuels DFT BH-LYP/TZVP, respectivement en utilisant la méthode COSMO.

7. Procédé selon l'une des revendications 1 à 6, **caractérisé en ce qu'**à l'étape e), l'erreur systématique est supposée être égale à +0,7 électronvolt.

8. Procédé selon l'une des revendications 1 à 6, **caractérisé en ce qu'**à l'étape e), l'erreur systématique est supposée être égale à +0,56 électronvolt.

**Figur 1:**

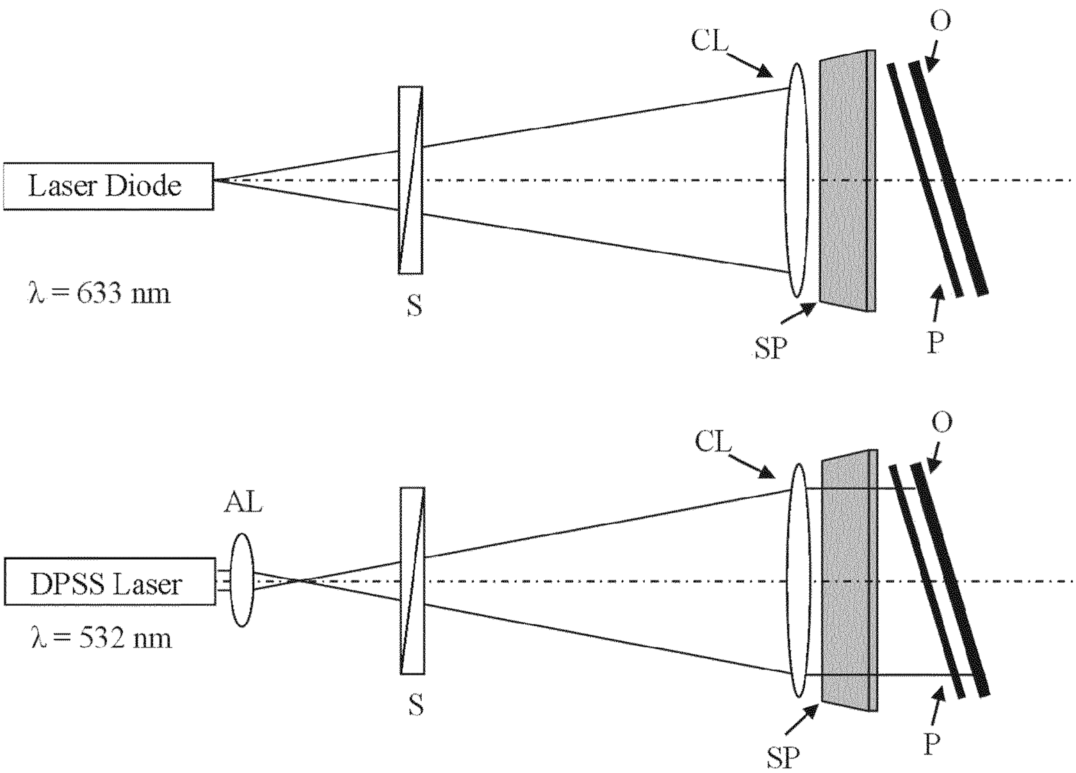


Figur 1 zeigt die Messanordnung zur Prüfung der holographischen Eigenschaften für Wellenlängen von 633 und 532 nm.

**Figur 2:**

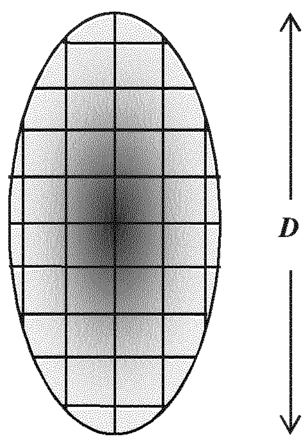


Figur 2 zeigt die elliptische Form eines unter Figur 1 geschriebenen Hologramms

Figur 3:

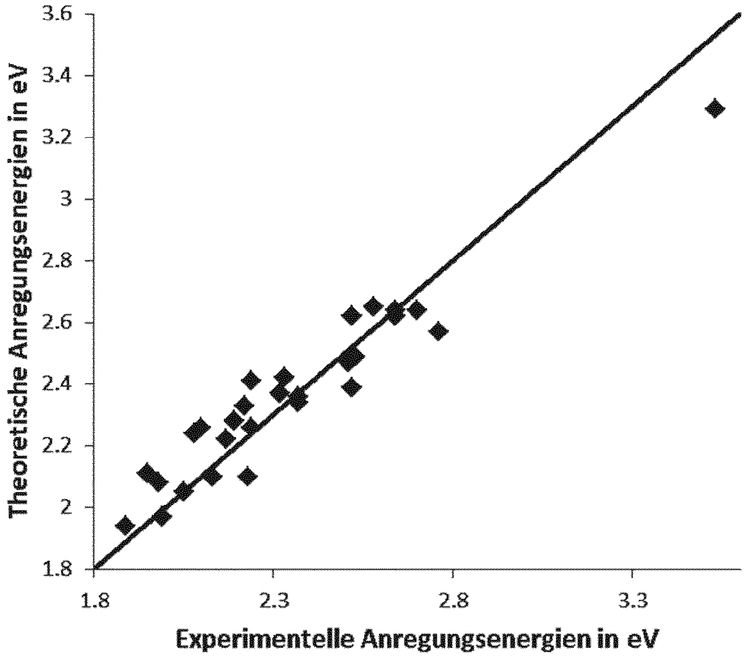


Figur 3 zeigt die Auftragung der theoretischen Anregungsenergien über die experimenteller Anregungsenergien der betrachteten Beispielfarbstoffe F1 – F28.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2008125229 A1
- US 2012237856 A
- EP 0671393 A
- WO 9514689 A
- US 3573289 A
- EP 58863 A
- DE 883025
- EP 1253148 A


### In der Beschreibung aufgeführte Nicht-Patentliteratur


- **P. HARIHARAN.** Optical Holography. Cambridge University Press, 1996
- **T. HELGAKER ; P. J¢RGENSEN ; J, OLSEN.** Molecular Electronic-Structure Theory. Wiley, 2000
- **R. G. BAK ; W. YANG.** Density-Functional Theory of Atoms and Molecules. Oxford University Press, 1989
- **A. KLAMT ; G. SCHÜÜRMANN.** *J. Am. Chem. Soc. Perkin Trans II,* 1993, 799
- **W. KOCH ; M. C. HOLTHAUSEN.** A Chemist's Guide to Density Functional Theory. Wiley-VHC
- Time-Dependent Density Functional Response Theory for Molecules. **M. E. CASIDA.** Recent Advances in Density Functional Methods. World Scientific, 1995, vol. 1, 155-192
- **L. GOERIGK ; S. GRIMME.** *J. Chem. Phys.,* 2010, vol. 132, 184103
- **D. REHM ; A. WELLER.** *Ber. Bunsenges. Physik. Chem.,* 1969, vol. 73, 834
- **A. D. BECKE.** *Phys. Rev. A,* 1998, vol. 38, 3098
- **J. PERDEW.** *Phys. Rev. B,* 1986, vol. 33, 8822
- **S. H. VOSKO ; L. WILK ; M. NUSAIR.** *Can. J. Phys.,* 1980, vol. 58, 1200
- **A. SCHÄFER ; C. HUBER ; R. AHLRICHS.** *J. Chem. Phys.,* 1994, vol. 100, 5829
- **A. KLAMT ; G. SCHÜÜRMANN.** *J. Am. Chem. Soc. Perkin Trans II,* 1993, vol. 799
- **A. D. BECKE.** *J. Chem. Phys.,* 1993, vol. 98, 1372
- *Synthesis,* 1999, 2103
- *J. Chem. Soc.,* 1938, 1454
- *Proceedings of the Imperial Academy (Tokyo),* 1932, vol. 8, 421
- *Chem. Zentralbl.,* 1934, vol. 105, 2227